# EUROPÄISCHE PATENTSCHRIFT

(11) **EP 2 387 945 B1**
(45) Veröffentlichungstag und Bekanntmachung des Hinweises auf die Patenterteilung: **02.12.2015**
(21) Anmeldenummer: 10163172.9
(22) Anmeldetag: 18.05.2010
(51) Int. Cl.: A61B 6/03, A61B 6/00, A61B 6/14, A61B 6/02, G06T 5/50, G06K 9/54, G06T 7/00, G06T 11/00, H04N 5/32

(54) **Verfahren und Vorrichtung zur digitalen Volumentomographie**
Method and device for digital volumetric tomography
Procédé et dispositif de tomographie volumétrique numérique

(43) Veröffentlichungstag der Anmeldung: 23.11.2011
(73) Patentinhaber: White Lion Technologies AG, 81377 München (DE)
(72) Erfinder: Baumgartner, Armin, 80995 München (DE)
(74) Vertreter: Ganahl, Bernhard

(56) Entgegenhaltungen:
- EP-A1- 1 882 449
- WO-A2-2007/100550
- US-A1- 2007 242 794
- US-A1- 2008 044 073
- US-B2- 7 471 768

## Beschreibung

Die vorliegende Erfindung betrifft ein Verfahren und eine Vorrichtung zur digitalen Volumentomographie.

Die digitale Volumentomographie (DVT) ist ein dreidimensionales, zahnärztliches bildgebendes Tomographie-Verfahren (CT), bei dem Röntgenstrahlen zum Einsatz kommen. Ähnlich wie bei der Computertomographie oder der Magnetresonanztomographie (MRT) ermöglicht die DVT die Erzeugung von Schnittbildern. Ein digitaler Volumentomograph besitzt eine um 180 oder 360 Grad rotierbare Röntgenröhre und einen zweidimensionalen Detektor (üblicherweise CCD-Detektor), der die gemessene Strahlung in Bilder umwandelt. Bei der Bilderstellung rotieren Röntgenröhre und Detektor um den fixierten Patienten. Dabei wird pro Winkeleinheit (z.B. je Grad) je ein zweidimensionales Summations-Einzelbild erstellt und aus den gewonnenen Bildern ein dreidimensionales Modell rekonstruiert. Daraus können Schnittbilder in allen Raumebenen sowie dreidimensionale Ansichten errechnet werden.

Ein handelsüblicher CCD-Chip kann z.B. bis zu 16000 Graustufen darstellen; mit neueren, weiter entwickelten Flächendetektoren sind sogar bis zu 64000 Graustufen möglich. Diese Datenmenge ist bei einem DVT-Gerät kaum handhabbar, da für jedes dreidimensionale Bild bis zu 500 oder mehr Einzelaufnahmen angefertigt werden. Aus diesem Grund werden bei DVT-Aufnahmen nur beispielsweise 4096 Graustufen ausgenutzt. Ein Spiral-CT nutzt beispielsweise 16000 Grauwerte; hier ist aber aufgrund des Funktionsprinzips einer Zeilenabtastung die Gesamtdatenmenge geringer.

Ein DVT-Gerät erzeugt zur Berechnung dreidimensionaler Strukturen zweidimensionale Bilder als Datensatz, während die Bildgebung eines CT-Geräts auf einer eindimensionalen Detektion beruht. Bezüglich der Strahlenexposition liegt die DVT unter der einer konventionellen Spiral-CT, was den Einsatz in einer Zahnarztpraxis durch den Zahnarzt ermöglicht (im Gegensatz zu einem CT-Gerät). Eine DVT-Rekonstruktion zeigt weniger Artefakte durch metallische Restaurationen in den zahntragenden Kieferbereichen als ein CT. Allerdings ist mittels DVT bisher keine Bildgebung unterhalb des Kopfes möglich, so dass die DVT auf die Anwendung in der Hals-, Nasen und Ohrenheilkunde (Nebenhöhlen, Mittelohr und Kiefergelenk) sowie Zahnmedizin bzw. Mund-Kiefer-Gesichtschirurgie beschränkt ist.

Die DVT wird in der Zahnheilkunde vornehmlich zur Planung von Operationen und dem Setzen von Implantaten verwendet. In der HNO-Heilkunde wird sie ebenfalls zur Diagnostik und vor Operationen im Bereich der Nasennebenhöhlen herangezogen. Derzeit kann die DVT noch nicht die Diagnostik von Frakturen, etc. ersetzen; hierfür kommen nach wie vor konventionelle Röntgenaufnahmen oder die Computertomographie zum Einsatz.

Grundsätzlich ist es bekannt, dass Röntgenröhren mit unterschiedlichen Frequenzbereichen oder Frequenzbändern, also unterschiedlichen Energien arbeiten können. Es ist möglich, durch Änderung der Anodenspannung etwa im Bereich zwischen 50 und 120 kV das von der Röntgenröhre abgegebene Frequenzspektrum zu steuern. Dies wird beispielsweise ausgenutzt, um unterschiedliche Gewebearten mit optimalem Kontrastverhältnis darstellen zu können. So ist es z.B. bekannt, dass verhältnismäßig niederfrequente Strahlung für weiches Gewebe wie Muskeln, Fett, Bindegewebe etc. einen guten Kontrast ergibt, während sie in dichteren Strukturen wie etwa Knochen nahezu vollständig "hängen" bleibt. Dagegen können Knochen mit verhältnismäßig hochfrequenter Strahlung gut dargestellt werden, während diese Strahlung weicheres Gewebe nahezu unbeeinflusst durchdringt. Röntgen- und DVT-Geräte sind so ausgerüstet, dass das Frequenzband oder die Energie der verwendeten Röntgenstrahlung eingestellt werden kann.

Aus der JP 2007-319575 (A) ist es bekannt, ein tomographisches Bild eines Objekts dadurch herzustellen, dass während eines Abtastvorgangs die Amplitude der Anodenspannung oder des Röhrenstroms von Abtastimpuls zu Abtastimpuls zwischen zwei oder drei Werten wechselt. Dabei wird vorgeschlagen, beispielsweise durch Ändern der Belichtungszeiten bei jedem Abtastimpuls im Wesentlichen die gleiche Photonenmenge auf das Abtastobjekt abzugeben.

In T. Freund, Methodische und klinische Evaluation eines modernen Flachbettscanners und des Dual Energy Verfahrens, 2006, Dissertation der Medizinischen Fakultät Charite der Humboldt-Universität zu Berlin wird beschrieben, wie zwei Röntgenstrahlen unterschiedlicher Energie auf die Knochen eines Patienten gerichtet werden, die beiden Bilder voneinander subtrahiert werden und ein Knochenbild, in dem die Weichteilstrukturen mathematisch herausgerechnet sind, und ein Weichteilbild, in dem die knöchernen Strukturen mathematisch herausgerechnet sind, entstehen.

Eine Aufgabe der Erfindung besteht darin, bestehende Verfahren und Vorrichtungen zur digitalen Volumentomographie zu verbessern. Eine Aufgabe besteht insbesondere, aber nicht ausschließlich, darin, die Bildqualität bei der digitalen Volumentomographie ohne Erhöhung, vorzugsweise bei gleichzeitiger Verringerung der Strahlenbelastung zu verbessern. Dabei kann sich die Verbesserung der Bildqualität beispielsweise im Kontrast, der Auflösung oder der Reduktion von Artefakten zeigen. Insbesondere ist es wünschenswert, ein Röntgengerät der vorgenannten Art derart weiterzubilden, dass die Bildqualität ohne Erhöhung der Strahlenbelastung verbessert, vorzugsweise die Strahlenbelastung noch weiter verringert werden kann.

Die Erfindung weist zur Lösung dieser Aufgabe die im Patentanspruch 1 oder 11 angegebenen Merkmale auf. Vorteilhafte Ausgestaltungen hiervon sind in den weiteren Ansprüchen angegeben.

Dokument EP1882449 A1 offenbart ein Verfahren und eine Vorrichtung nach dem Oberbegriff von Ansprüchen 1 und 11.

Nach einem Gesichtspunkt der vorliegenden Erfindung werden in einem Verfahren zur digitalen Volumentomographie an einem Objekt, bei welchem eine Röntgenfunktionseinheit mit einer Röntgenquelle und einer digitalen, zweidimensionalen Röntgendetektoreinrichtung und einem dazwischen sich erstreckenden Raumvolumen, in welchem sich das Objekt befindet, gedreht wird und dabei einen vorbestimmten Winkelbereich überstreicht, die Röntgenquelle in diskreten, vorbestimmten Zeit- oder Winkelabständen angesteuert wird, um Röntgenstrahlen auszusenden, die durch die Detektoreinrichtung aufgefangenen Signale in Zuordnung zu der jeweiligen Winkellage als zweidimensionale Einzelbilder gespeichert werden, und aus den Einzelbildern eine dreidimensionale Darstellung des abgetasteten Raumvolumens rekonstruiert wird, Röntgenstrahlen wenigstens zweier unterschiedlicher Frequenzbereiche verwendet, wobei ein Grauwertabgleich während einer Bildbearbeitung oder einer Rekonstruktion durchgeführt wird, um Unterschiede in der Gesamthelligkeit von in unterschiedlichen Frequenzbereichen aufgenommenen Bildern auszugleichen.

Bei Volumentomographen mit Flächendetektor ist konstruktionsbedingt ein geringerer Bildkonstrast als etwa bei einem Spiraltomographen (CT) möglich. Werden bei der Röntgenabtastung Röntgenstrahlen unterschiedlicher Frequenzbereiche, also unterschiedlicher Energien verwendet, führen die unterschiedlichen Bestrahlungsleistungen zu Unterschieden in der Gesamthelligkeit des aufgenommenen Bildes. Eine Zusammenführung der Bildinformationen der mit unterschiedlicher Bestrahlungsleistung aufgenommenen Bilder würde ohne weitere Maßnahmen zu unbefriedigenden Ergebnissen führen. Wenn ein Grauwertabgleich während der Bildbearbeitung oder Rekonstruktion durchgeführt wird, können Unterschiede in der Gesamthelligkeit von in unterschiedlichen Frequenzbereichen aufgenommenen Bildern eliminiert werden. Durch Verwendung von Röntgenprimärdaten mit unterschiedlichen Röntgenspektren unter Ausgleich der Gesamthelligkeitsunterschiede in den beiden Röntgenspektren durch einen Grauwertabgleich kann auch der Bildkontrast gegenüber herkömmlichen DVT-Verfahren verbessert werden.

Bei der oben diskutierten Röntgenvorrichtung gemäß der JP 2007-319575 A wird ein tomographisches Bild eines Objektes hergestellt, wobei während eines Abtastvorganges die Amplitude der Anodenspannung oder des Röhrenstroms von Abtastimpuls zu Abtastimpuls zwischen zwei oder drei Werten wechselt. Bei dieser bekannten Vorrichtung werden die einzelnen Abtastimpulse so eingestellt, dass ein jedes 2D-Bild mit möglichst der gleichen Energiedosis belichtet wird. Dies kann beispielsweise durch Verändern der Belichtungszeit oder durch Verändern des Stromes zum Betreiben der Röntgenröhre eingestellt werden. Hierdurch werden die einzelnen Bilder vergleichbar und können zu einem Volumenbild rekonstruiert werden. Hiermit wird das Signal-Rausch-Verhältnis verbessert.

Die vorliegende Erfindung unterscheidet sich von diesem bekannten Verfahren dadurch, dass ein Grauwertabgleich während der Rekonstruktion oder der Bildbearbeitung der mit unterschiedlichen Frequenzen aufgenommenen Bildern durchgeführt wird. Hierdurch ist es nicht notwendig die Energiedosis der einzelnen 2D-Bilder aneinander anzugleichen. Die einzelnen 2D-Bilder werden vielmehr in der Regel mit unterschiedlicher Energiedosis aufgenommen, wobei die Energiedosis so eingestellt ist, dass ein optimaler Kontrast im jeweiligen Bild erhalten wird. Durch den aus dem Stand der Technik bekannten Abgleich der Energiedosis ist der Kontrast in den einzelnen 2D-Bildern nicht optimal, das heißt, dass entweder Weichteile zu stark durchleuchtet werden, so dass sie nicht mehr sichtbar sind, oder die Intensität der Strahlung so gering ist, dass härtere Teile nicht mehr ausreichend durchdrungen werden. Ein solcher Grauwertabgleich führt nicht unbedingt zu einer Erhöhung des Signal-Rausch-Verhältnisses, sondern zu einer Verbesserung des Dynamikbereiches. Durch die Erhöhung der Dynamik werden in einem Bild sowohl Weichteile als auch Knochenstrukturen gut erkennbar. Die Qualität der dem erfindungsgemäßen Verfahren erzeugten Bilder entspricht der Qualität von mit herkömmlichen CT-Vorrichtungen erzeugten Bildern, wobei bei der Volumentomographie die Strahlenbelastung wesentlich geringer als bei der mit der Computertomographie erzeugten Bildern ist. Zudem sind Geräte zur Durchführung der Volumentomographie wesentlich günstiger als Geräte zur Durchführung der Computertomographie.

Vorzugsweise werden die einzelnen 2D-Bilder mit einer sich jeweils erheblich unterscheidenden Energiedosis belichtet. Die Energiedosen sind vorzugsweise etwa proportional zur Frequenz beziehungsweise Energie der einzelnen Röntgenquanten. Je nach zu untersuchendem Objekt können die Energiedosen entsprechend frei angepasst werden, um das jeweilige Objekt mit maximalem Kontrast zu erfassen.

Mit der Erfindung wird somit ein völlig anderer Weg als im Stand der Technik eingeschlagen, nämlich nicht die Grauwerte durch Einstellen der Energiedosen bei der Belichtung der einzelnen 2D-Bilder einander anzupassen, sondern den Grauwertabgleich bei der Rekonstruktion oder bei einer der Rekonstruktion vorhergehenden digitalen Bildbearbeitung durchzuführen. Hierdurch beruht die Rekonstruktion auf wesentlich mehr Information, so dass die Qualität des rekonstruierten Bildes erheblich gesteigert werden kann.

Somit wird mit der vorliegenden Erfindung mit einfachen Mitteln die Qualität der Bilder erheblich gesteigert.

Der vorbestimmte Winkelbereich beträgt vorzugsweise 360 oder 180 °, kann aber grundsätzlich je nach diagnostischen Bedürfnissen beliebig wählbar sein.

Ein Frequenzbereich im Sinne der vorliegenden Erfindung kann durch das Frequenzspektrum, eine Grenzfrequenz, vorzugsweise untere Grenzfrequenz, ein Frequenzband, eine Einzelfrequenz oder eine Auswahl von Einzelfrequenzen, eine Photonenenergie, eine Beschleunigungsspannung, eine Heizspannung oder einen Röhrenstrom oder mehreren von diesen definierbar sein.

Für die Zwecke dieser Anmeldung umfasst der Begriff der Röntgenstrahlung alle elektromagnetischen Strahlen, welche geeignet sind, Bildaufnahmen von räumlichen Strukturen herzustellen, indem sie mit unterschiedlichen Substanzen in unterschiedlichem Maße wechselwirken. Unter einem Wechselwirken wird im Sinne der Erfindung eine Absorption, Streuung, Reflektion oder sonstige Beeinflussung der Photonen der Röntgenstrahlung verstanden.

In einer bevorzugten Ausführungsform wird der vorbestimmte Winkelbereich in wenigstens zwei Durchgängen von der Röntgenfunktionseinheit überstrichen, wobei bei jedem Durchgang ein anderer Frequenzbereich der Röntgenquelle verwendet wird, um Einzelbilder zu erzeugen, und wobei die Einzelbilder jedes Durchgangs zu einer räumlichen Darstellung rekonstruiert werden, wobei ein Grauwertabgleich zwischen den räumlichen Darstellungen auf der Grundlage eines mittleren Grauwerts der Voxel jeder räumlichen Darstellung durchgeführt wird, und wobei die räumlichen Darstellungen nach dem Grauwertabgleich überlagert werden, um eine einzige räumliche Darstellung zu erzeugen. Bei dieser Ausführungsform kann ein eingestellter Frequenzbereich über einen längeren Zeitraum beibehalten werden, was geringere Anforderung an die Flexibilität und Ansteuerung der Röntgenquelle stellt. Die Überlagerung kann mit Hilfe eines Stitching-Verfahrens durchgeführt werden. Unter einem Durchgang wird im Sinne der Erfindung ein einmaliges Überstreichen des vorbestimmten Winkelbereich verstanden.

Vorzugsweise wechselt die Drehrichtung nach jedem Überstreichen des vorbestimmten Winkelbereichs. Dabei kann in der weiteren Bildverarbeitung und Rekonstruktion vorteilhaft ausgenutzt werden, dass am Wendepunkt der Drehrichtung zwei Bilder mit unterschiedlichem Frequenzspektrum, aber gleicher Winkellage vorliegen. Die mechanische Belastung der drehenden Teile und der Energieverbrauch für den Antrieb kann verringert werden, da der insgesamt zu fahrende Drehweg verringert werden kann.

Besonders bevorzugt wird der Grauwertabgleich anhand eines Referenzkörpers durchgeführt, der in der Nähe oder innerhalb des Objekts im Strahlengang der Röntgenfunktionseinheit angeordnet ist. Der Referenzkörper ist vorzugsweise aus einem Kunststoff hergestellt, der mit einer Röntgenkontrastsubstanz dotiert oder beschichtet sein kann. Als Röntgenkontrastsubstanz kann Bariumsulfat, Eisen, Titan oder dergleichen, vorzugsweise als Mikro- oder Nanopartikel, eingesetzt werden. Die Dotierung oder Beschichtung ist vorzugsweise so gewählt, dass eine Wechselwirkung von Röntgenstrahlung mit dem Referenzkörper einer Wechselwirkung mit einer Materialart, welche in dem Objekt enthalten ist, ähnlich ist.

In einer alternativen bevorzugten Ausführungsform wird der vorbestimmte Winkelbereich nur ein einziges Mal durchlaufen, wobei der Fequenzbereich der Röntgenquelle nach jedem Winkelinkrement gewechselt wird, wobei ein Grauwertabgleich zwischen benachbarten Einzelbildern auf der Grundlage eines mittleren Grauwerts der Pixel jedes Einzelbildes durchgeführt wird. Es ist bei dieser Ausführungsform nur ein Umlauf erforderlich. Hierdurch wird die Rekonstruktion der Bilder erheblich vereinfacht, da im Wesentlichen die bisher verwendeten Auswertungsverfahren verwendet werden können. Eigenbewegungen des Objekts können leichter kompensiert werden. Die Strahlenexposition kann gegenüber der Ausführungsform mit zwei Umläufen verringert werden.

Besonders bevorzugt wird der Grauwertabgleich anhand eines Referenzkörpers durchgeführt, der in der Nähe der Röntgendetektoreinrichtung mitdrehend im Strahlengang der Röntgenfunktionseinheit angeordnet ist. Der Referenzkörper ist vorzugsweise aus einem Kunststoff hergestellt, der mit einer Röntgenkontrastsubstanz dotiert oder beschichtet sein kann. Besonders bevorzugt ist der Referenzkröper ein beschichtets Folienstück, etwa Foliendreieck, das auf der Oberfläche der Röntgendetektoreinrichtung im Randbereich, möglichst ohne das Objekt zu verdecken, aufgebracht ist. Als Röntgenkontrastsubstanz kann Bariumsulfat, Eisen, Titan oder dergleichen, vorzugsweise als Mikro- oder Nanopartikel, eingesetzt werden. Die Dotierung oder Beschichtung ist vorzugsweise so gewählt, dass eine Wechselwirkung von Röntgenstrahlung mit dem Referenzkörper einer Wechselwirkung mit einer Materialart, welche in dem Objekt enthalten ist, ähnlich ist.

Nach einem zweiten Gesichtspunkt der vorliegenden Erfindung weist eine Vorrichtung zur digitalen Volumentomographie eine drehbare Röntgenfunktionseinheit mit einer Röntgenquelle und einer digitalen Detektoreinrichtung, wobei die Röntgenquelle eingerichtet und angepasst ist, Röntgenstrahlen verschiedener Frequenzbereiche abzugeben, und eine Steuereinrichtung, die eingerichtet und angepasst ist, eine Antriebseinrichtung anzusteuern, um die Röntgenfunktionseinheit zu drehen und eine vorgegebene Winkellage einzustellen, die Röntgenquelle in diskreten Winkel- oder Zeitinkrementen mehrmals anzusteuern, um Röntgenstrahlung abzugeben, und die von der Detektoreinrichtung aufgefangenen Signale als zweidimensionale Einzelbilder zu speichern und einer bestimmten Winkellage zuzuordnen, und aus den Einzelbildern eine dreidimensionale Darstellung zu berechnen, und wobei die Vorrichtung eingerichtet und angepasst ist, eines der vorstehend beschriebenen Verfahren durchzuführen.

Die Verbindung zwischen der Röntgenquelle, der Röntgendetektoreinrichtung und dem Drehantrieb einerseits und der Steuerungseinrichtung andererseits kann drahtgebunden und/oder drahtlos, beispielsweise, aber nicht nur, über Bluetooth, WLAN, eine Infrarotverbindung und/oder dergleichen herstellbar sein.

In einer bevorzugten Ausführungsform kann die Röntgenquelle zwei Röntgenröhren aufweisen, die in unterschiedlichen Frequenzbereichen arbeiten und die wahlweise aktivierbar sind.

In einer alternativen Ausführungsform kann die Röntgenquelle eine breit- oder mehrbandige Röhre aufweisen sowie eine Frequenzselektionseinrichtung, die ausgelegt und eingerichtet ist, unterschiedliche Frequenzen wahlweise durchzulassen.

Vorzugsweise weist die Frequenzselektionseinrichtung wenigstens einen Bragg-Kristall auf, der mit veränderbarer Lage in dem Strahlengang platziert ist und/oder, falls mehrere Bragg-Kristalle vorhanden sind, die wahlweise in dem Strahlengang platzierbar sind.

Die vorstehende Aufgabe, die Merkmale und Vorteile nach der vorliegenden Erfindung können unter Berücksichtigung der folgenden, detaillierten Beschreibung der bevorzugten Ausführungsformen der vorliegenden Erfindung und unter Bezugnahme auf die zugehörigen Zeichnungen besser verstanden werden.

Die Erfindung wird im folgenden anhand der Zeichnungen näher erläutert. Diese zeigen in:
Fig. 1 eine schematische Gesamtdarstellung eines DVT-Diagnosesystems nach der Erfindung;
Fig. 2 eine perspektivische Darstellung eines DVT-Diagnosegeräts in dem Diagnosesystem von Fig. 1;
Fig. 3 eine schematische Seitenansicht eines C-Arms des DVT-Diagnosegeräts von Fig. 2;
Fig. 4 eine Horizontalschnittansicht der Anordnung von Fig. 3 auf der Höhe einer Mittellinie eines Strahlengangs einer Röntgenquelle in Blickrichtung eines Pfeils "IV" in Fig. 3;
Fig. 5 eine schematische Darstellung des Aufbaus einer Röntgenquelle und eines Röntgenvorschaltgeräts in dem Gerät von Fig. 2;
Fig. 6 eine schematische Darstellung eines Aufbau eines CCD-Sensors in dem Gerät von Fig. 2;
Fig. 7 eine schematische Darstellung einer Zusammenschaltung funktionaler Einheiten des DVT-Systems von Fig. 1;
Fig. 8 ein Ablaufdiagramm zur Darstellung einer beispielhaften Routine zur Anfertigung einer DVT-Aufnahme mit einer gleich bleibenden, vorgebbaren Frequenz;
Fig. 9 ein Ablaufdiagramm zur Darstellung einer beispielhaften Routine zur Anfertigung einer DVT-Aufnahme unter Verwendung zweier vorgebbarer Frequenzen gemäß einer Ausführungsvariante der vorliegenden Erfindung;
Fig. 10 ein Ablaufdiagramm zur Darstellung einer beispielhaften Routine zur Normalisierung von Grauwerten in dieser Ausführungsvariante der vorliegenden Erfindung;
Fig. 11 zeigt eine Anordnung eines C-Arms und eines Diagnosekörpers mit einem Referenzkörper gemäß einer Ausführungsvariante der vorliegenden Erfindung;
Fig. 12 ein Ablaufdiagramm zur Darstellung einer beispielhaften Routine zur Normalisierung von Grauwerten in einer weiteren Ausführungsvariante der vorliegenden Erfindung;
Fig. 13A und Fig. 13B ein Ablaufdiagramm zur Darstellung einer beispielhaften Routine zur Anfertigung einer DVT-Aufnahme unter Verwendung zweier vorgebbarer Frequenzen gemäß einer weiteren Ausführungsvariante der vorliegenden Erfindung, wobei ein Übergang von Fig. 13A zu Fig. 13B an einer Verknüpfungsstelle erfolgt;
Fig. 14 ein Ablaufdiagramm zur Darstellung einer beispielhaften Routine zur Normalisierung von Grauwerten in dieser Ausführungsvariante der vorliegenden Erfindung;
Fig. 15 ein Ablaufdiagramm zur Darstellung einer alternativen Routine zur Normalisierung von Grauwerten in dieser Ausführungsvariante der vorliegenden Erfindung;
Fig. 16 eine Anordnung eines CCD-Sensors mit einem Referenzobjekt gemäß einer weiteren Ausführungsvariante der vorliegenden Erfindung;
Fig. 17 eine schematische Darstellung einer Röntgenquelle mit zwei Röntgenröhren gemäß einer weiteren Ausführungsvariante der vorliegenden Erfindung;
Fig. 18 eine weitere schematische Darstellung einer Röntgenquelle mit zwei Röntgenröhren gemäß einer weiteren Ausführungsvariante der vorliegenden Erfindung;
Fig. 19 eine schematische Darstellung einer Röntgenquelle mit zwei Bragg-Kristallen gemäß einer weiteren Ausführungsvariante der vorliegenden Erfindung;
Fig. 20 eine schematische Darstellung einer Röntgenquelle mit mehreren Bragg-Kristallen gemäß einer weiteren Ausführungsvariante der vorliegenden Erfindung; und
Fig. 21 eine Ansicht der Röntgenquelle von Fig. 20 in Richtung eines Pfeils "XXI" in Fig. 20.

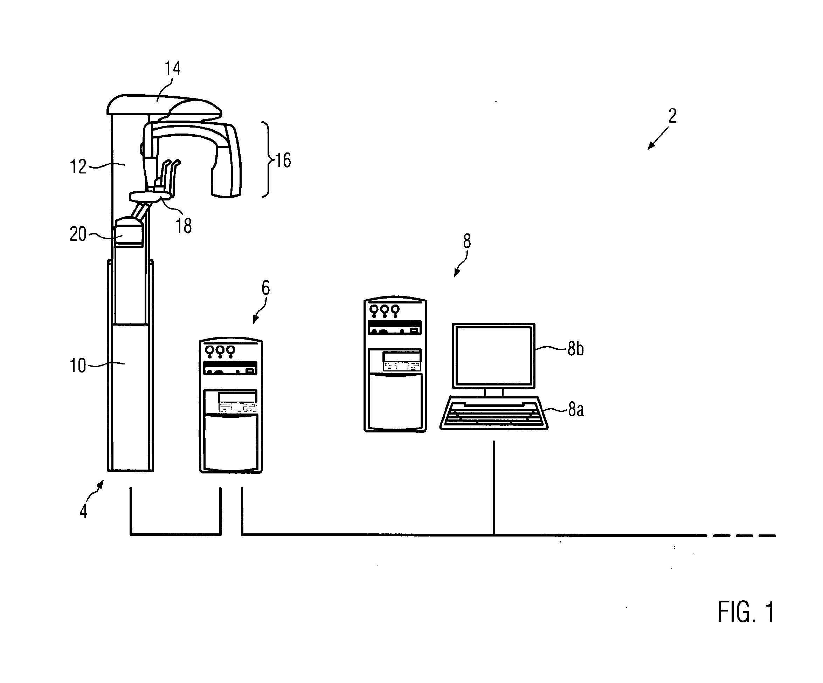

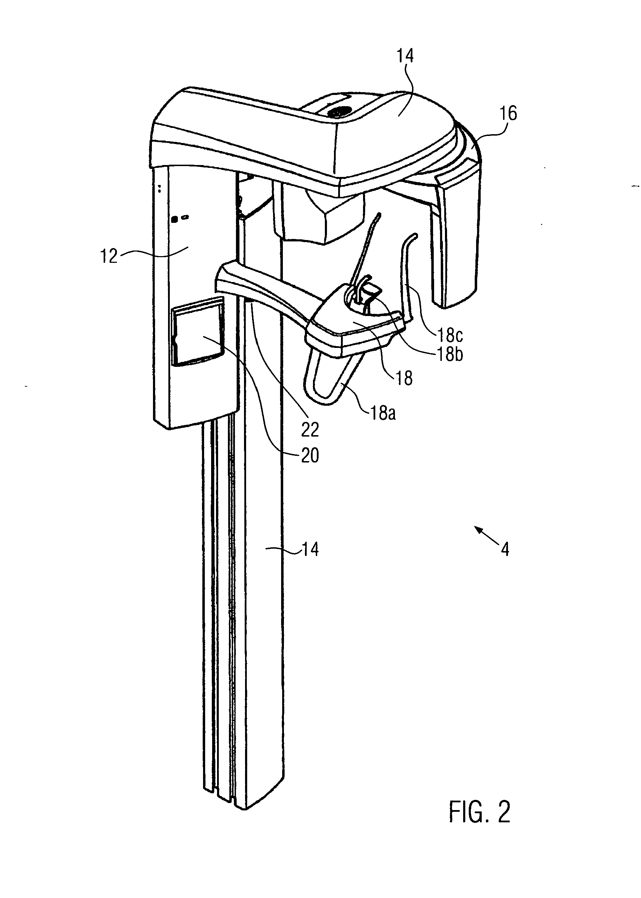

Fig. 1 zeigt ein DVT-Diagnosesystem in einer schematischen Gesamtdarstellung und in Fig. 2 ist ein DVT-Diagnosegerät des Diagnosesystems von Fig. 1 perspektivisch dargestellt. Das äußere Erscheinungsbild entspricht einem Stand der Technik, wie er in öffentlich zugänglichen Produktunterlagen der Firma Planmeca entnehmbar ist. Die Geräte sind gemäß der vorliegenden Erfindung wenigstens in ihrem inneren Aufbau bzw. ihrer Ansteuerung an die Durchführung eines erfindungsgemäßen Verfahrens angepasst.

Gemäß der Darstellung in Fig. 1 weist ein DVT-Diagnosesystem 2 ein DVT-Gerät 4, einen Server 6 und eine Arbeitsstation 8 auf. Das DVT-Gerät 4, der Server 6 und die Arbeitsstation 8 sind über ein Netzwerk miteinander verbunden, das in der Figur nur angedeutet ist. Die Arbeitsstation 8 weist eine Tastatur 8a und einen Bildschirm 8b auf. Das Netzwerk kann ganz oder in Teilen kabelgebundene oder kabellose, peer-to-peer-basierte oder client/server-basierte, stern- oder ringtopographische Elemente aufweisen, über TCP/IP, WLAN, Bluetooth, Infrarot oder dergleichen Protokolle ausgestaltet sein. In dem Netzwerk können noch weitere Arbeitsstationen und Peripheriegeräte angeschlossen sein.

Das DVT-Gerät 4 weist eine fest stehende oder an einer Wand befestigte Säule 10 auf, an welcher ein Läufer 12 vertikal beweglich und antreibbar befestigt ist. An der Säule 10 kann ein Sitz (nicht näher dargestellt) befestigt sein, auf welchem eine Person Platz nehmen kann.

Ein Ausleger 14 ragt am oberen Ende des Läufers 12 horizontal ab. Der Ausleger 14 kann, muss aber nicht, schwenkbar sein und/oder ein Gelenk aufweisen oder auf andere Weise in der Auslegerlänge änderbar sein. Am Ende des Auslegers 14 ist ein sog. C-Arm 16 drehbar angebracht. Am horizontalen Teil des Läufers 12 ist ferner ein Positionierungstisch 18 angebracht.

Gemäß der Darstellung in Fig. 2 weist der Positionierungstisch 18 einen Handgriffbügel 18a, eine Kinnauflage 18b und eine Stirn- oder Schläfenstütze 18c auf. Ferner sind eine an dem Läufer 12 angebrachte Bedienkonsole 20 und ein Not-Aus-Schalter 22 dargestellt. Ein an der Rückseite des Läufers 12 angeordneter Ein-Aus-Schalter ist in der Figur nicht sichtbar.

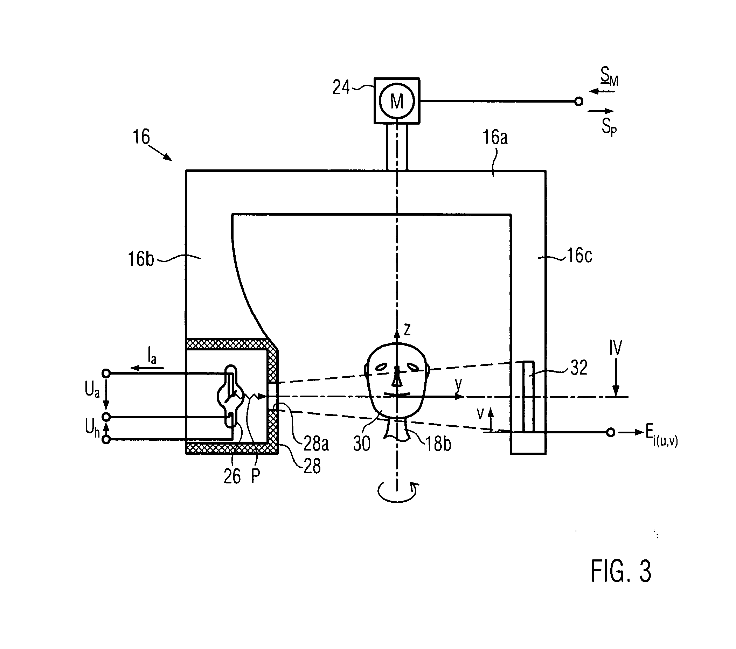

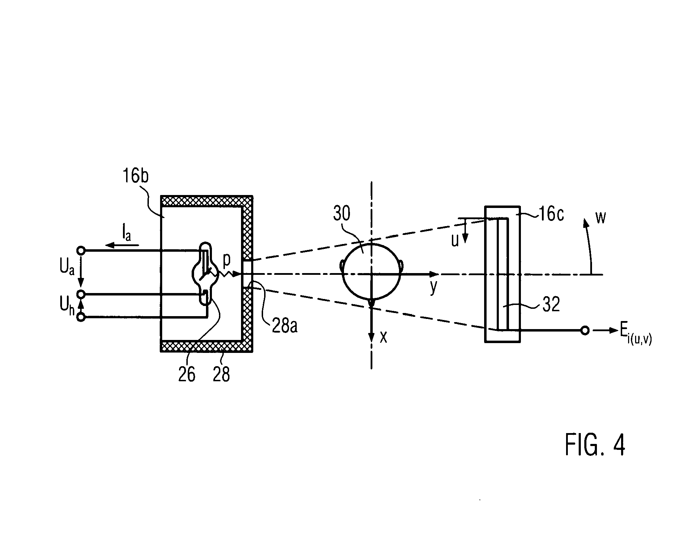

Fig. 3 zeigt den C-Arm 16 von der Seite gesehen in einer schematischen Darstellung mit einigen sichtbaren Einbauteilen und einem abzutastenden Objekt (Diagnoseobjekt), und Fig. 4 zeigt einen Horizontalschnitt der Anordnung von Fig. 3 auf der Höhe einer Mittellinie eines Strahlengangs in Blickrichtung eines Pfeils "IV in Fig. 3. Die Figuren zeigen die Anordnung in einer Ausgangsstellung, also vor Beginn einer Diagnosesitzung.

Gemäß der Darstellung in Fig. 3 weist der C-Arm 16 eine Basis 16a, einen ersten Schenkel 16b und einen zweiten Schenkel 16c auf. Der erste Schenkel 16b und der zweite Schenkel 16c ragen jeweils von den gegenüberliegenden Seiten der Basis 16a ab, sodass insgesamt in etwa die Querschnittsform eines "C" entsteht. Der erste Schenkel trägt eine Röntgeneinrichtung und wird in der weiteren Beschreibung auch als Belichtungsschenkel 16b bezeichnet. Der zweite Schenkel 16c weist eine Sensoreinrichtung auf und wird wird in der weiteren Beschreibung auch als Sensorschenkel 16c bezeichnet.

Die Basis 16a des C-Arms 16 ist mit einem Motor 24 verbunden und durch diesen antreibbar. Der Motor 24 ist z.B. ein Schrittmotor oder ein positionsgeregelter Servomotor. Der Motor 24 wird über ein von außen eingegebenes Motor-Ansteuerungssignal s_{M} angesteuert (alternativ kann auch ein Positions-Sollsignal eingegeben werden), und die jeweilige Drehlage des Motors 24 wird über ein Positionssignal s_{P} zurückgemeldet. (Im Fall eines Schrittmotors kann die Rückmeldung entfallen, eine Rückmeldung kann dennoch auch aus Sicherheitsgründen zur Fehlerkontrolle vorgesehen sein.) Ein ggf. noch vorhandenes Übersetzungs-, Untersetzungs- und/oder Umlenkgetriebe ist in der Figur nicht dargestellt.

Der Belichtungsarm 16b trägt eine Röntgenröhre 26. Die Röntgenröhre 26 wird über eine Heizspannung Uₕ und eine Anodenspannung Uₐ angesteuert, wobei in der Röhre ein Röhren- oder Anodenstrom lₐ fließt, wie weiter unten erläutert werden wird. Die Röntgenröhre 26 wird von einer Abschirmung 28 umgeben (die Abschirmung 28 umgibt die Röntgenröhre 26 allseits; ein rückwärtiger Teil ist in der Zeichnung aus Gründen der Deutlichkeit weggelassen). In der Abschirmung 28 ist auf der dem Sensorschenkel 16c zugewandten Seite ein Fenster (Blende) 28a eingelassen.

In der Figur ist der auf der Kinnauflage 18b ruhende Kopf eines Patienten als ein Diagnoseobjekt 30 dargestellt. Bei Betätigung der Röntgenröhre 26 sendet diese Röntgen-Photonen p aus, die durch das Fenster 28a der Abschirmung 28 fallen, den Raum, in welchem sich das Diagnoseobjekt 30 befindet, durchlaufen und auf der Seite des Sensorarms 16c auf einen CCD-Sensor 32 fallen. Der CCD-Sensor 32 wandelt die auf ihn einfallenden Röntgen-Strahlen (Photonen p) in ein zweidimensionales Bildsignal (Einzelbildsignal) Eᵢ(u,v) um und gibt diese aus. Die Argumente u und v entsprechen den diskreten Raumkoordinaten des CCD-Sensors 32; jedes Parameterpaar (u,v) entspricht somit einem Pixel des CCD-Sensors 32.

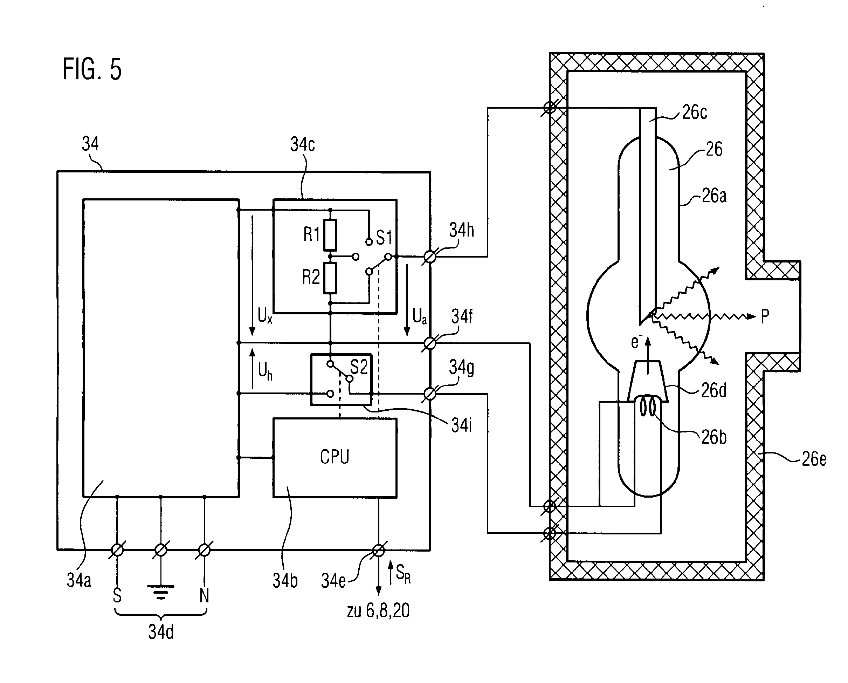

Der C-Arm 16 mit der Röntgenrohre 26 und dem CCD-Sensor 32 ist eine Röntgenfunktionseinheit im Sinne der Erfindung. Die Röntgenröhre 26 und ein Röntgenvorschaltgerät (vgl. Fig. 5) sind eine Röntgenquelle im Sinne der Erfindung. Der CCD-Sensor 32 ist eine Röntgendetektoreinrichtung im Sinne der Erfindung.

Die Pixelkoordinaten u,v des CCD-Sensors 32 und die Winkellage w des C-Arms können als ein Orts- oder Bildkoordinatensystem betrachtet werden.

Ein raumfestes oder zumindest apparatefestes, kartesisches Bezugskoordinatensystem ist wie folgt definiert. Die Drehachse des C-Arms 16 definiert eine raumfeste, vertikale Achse z. Das Diagnoseobjekt 30 ist so angeordnet, dass die Vertikalachse z in etwa durch sein Zentrum verläuft. Der Nullpunkt der Vertikalachse ist durch eine horizontale Ebene definierte, in welcher die Mittelllinie des Strahlengangs von der Röntgenquelle 26 durch das Fenster 28a zu dem CCD-Sensor 32 verläuft. Eine erste horizontale Raumrichtung y ist beliebig festlegbar; sie kann z.B. mit einer Erstreckungsrichtung des Auslegers zusammenfallen oder allgemein mit der Mittelllinie des Strahlengangs in einer Nullwinkellage des C-Arms 16 zusammenfallen. Eine zweite horizontale Raumrichtung x ergänzt die Raumrichtungen y, z zu einem kartesischen Koordinatensystem. Es versteht sich, dass die Definition des raumfesten Koordinatensystems nach den Gegebenheiten und Bedürfnissen der Einzelanwendung gewählt werden kann. Ohne Beschränkung der Allgemeinheit ist das Diagnoseobjekt 32 (der Kopf des Patienten) in der Ausgangsstellung so angeordnet, dass die Kopfseiten zu den Schenkeln 16b, 16c des C-Arms 16 weisen und die Blickrichtung der x-Achse entspricht.

Fig. 5 zeigt schematisch den Aufbau der Röntgenquelle 26 und eines Röntgenvorschaltgeräts 34.

Das Röntgenvorschaltgerät 34 weist eine Hochspannungsquelle 34a, eine zentrale Verarbeitungseinheit (CPU) 34b, ein Anodenschaltnetz 34c mit zwei verschiedenen Widerständen R1, R2 und einem Schalter S1, einen Netzanschluss 34d mit drei Polen (Phase L, Nullleiter N und Masse), einen Signaleingang 34e, einen Masseausgang 34f, einen Heizspannungsausgang 34g, einen Anodenspannungsausgang 34h und ein Kathodenschaltnetz 34i mit einem Schalter S2 auf. Der Netzanschluss 34d ist mit einem Stromversorgungsnetz verbindbar. Der Signaleingang 34e ist mit der Bedienkonsole 20 und über das in Fig. 1 skizzierte Netzwerk mit dem Server 6 und/oder der Arbeitsstation 8 verbindbar. Die Hochspannungsquelle 34a ist eingerichtet und ausgelegt, die über den Netzanschluss 34d anliegende Netzspannung in zwei Hochspannungspotentiale Uₕ, Uₓ umzuwandeln. Das Hochspannungspotential Uₓ liegt an einem Eingang des Anodenschaltnetzes 34c an. Ein Ausgang des Schaltnetzes 34c liegt am Anodenspannungsausgang 34h an. In dem Anodenschaltnetz 34c ist eine Spannungsteilerschaltung mit Widerständen R1 und R2, verwirklicht, wobei das Hochspannungspotential Uₓ oder eine Teilspannung hiervon mittels des Schalters S1 abgegriffen wird, das als ein Anodenspannungspotential Uₐ an einem Ausgang des Anodenschaltnetzes 34c und somit an dem Anodenspannungsausgang 34h anliegt. In einer weiteren Schaltstellung des Schalters S1 liegt der Ausgang des Anodenschaltnetzes 34c an Masse. Das Hochspannungspotential Uₕ liegt an dem Eingang des Kathodenschaltnetzes 34i an. Der Schalter S2 des Kathodenschaltnetzes 34i weist zwei Schaltstellungen auf, wobei in einer Schaltstellung der Ausgang an Masse liegt und in der anderen Schaltstellung der Eingang mit dem Ausgang verbunden ist und somit das Heizspannungspotential Uₕ als Heizspannung am Heizspannungsausgang 34g anliegt. Das Massepotential liegt am Masseausgang 34f an.

Anstelle des dreipoligen Netzanschlusses 34d kann auch ein fünfpoliger Netzanschluss mit drei Phasen oder ein Netzanschluss mit noch mehr Polen bzw. Phasen vorgesehen sein.

Die Röntgenquelle 26 weist einen evakuierten Kolben 26a, eine Kathode 26b und eine Anode 26c, einen Wehnelt-Zylinder 26d und eine Röhrenabschirmung 26e auf. Der Kolben 26a ist aus Metall oder Glas hergestellt. Die Anode 26b ist mit dem Anodenausgang 34h des Röntgenvorschaltgeräts 34 verbunden. Die Kathode 26c weist eine Heizwendel auf, deren Enden mit dem Heizspannungsausgang 34g bzw. dem Masseausgang 34f des Röntgenvorschaltgeräts 34 verbunden sind. Wenn die Kathode 26b von Strom bei anliegender Heizspannung Uₕ durchflossen und hierdurch erwärmt wird, werden Elektronen e frei, die unter der Wirkung der Anodenspannung Uₐ in Richtung auf die Anode 26c beschleunigt werden (die Anodenspannung wird auch als Beschleunigungsspannung bezeichnet). Der Wehnelt-Zylinder 26d ist zwischen der Kathode (Heizwendel) 26b und der Anode 26c angeordnet und dient der Ausrichtung der von der Kathode 26b abgegebenen Elektronen e in Richtung der Anode 26c. Die Elektronen e dringen in das Anodenmaterial ein, werden abgebremst und erzeugen unter anderem eine Röntgenbremsstrahlung, die im Bereich der DVT verwendet wird. Diese Strahlung wird in der Figur durch ein Photon p symbolisiert. Sie weist ein Spektrum mit einer unteren Grenzwellenlänge auf, deren Wert von der Anodenspannung Uₐ abhängt. Zwei andere Strahlungsarten, die diskrete Röntgenstrahlung (eine Röntgenstrahlung mit einem Linienspektrum) und die Lilienfeldstrahlung (eine Strahlung im Bereich sichtbaren Lichts) werden durch andere physikalische Effekte ebenfalls erzeugt, aber im Bereich der DVT nicht ausgenutzt. Wenn zur Beschreibung der Ausführungsformen Erfindung von Röntgenstrahlung die Rede ist, ist damit eine Röntgenbremsstrahlung gemeint. Die auf die Anode einfallenden Elektronen bewirken an der Anode einen Anodenstrom Iₐ.

Die Röntgenstrahlung p wird von der Anode 26c aus in einem großen Winkelbereich abgestrahlt, von welchem nur ein Anteil durch eine Öffnung in der Röhrenabschirmung 26e hindurchtritt und genutzt wird, während der Rest in der Röhrenabschirmung 26e abgefangen wird. Eine Kühlung der Röhre, die zur Abführung der hohen Wärmeverluste (eine Verlustrate von 99% ist ein üblicher Wert) vorhanden ist, ist in der Figur nicht näher dargestellt.

Es versteht sich, dass die Darstellung des Röntgenvorschaltgeräts 34 und der Röntgenröhre 26 sehr schematisch ist und auf die für das Verständnis der Erfindung hilfreichen Elemente beschränkt ist. Die Schaltnetze 34c, 34i können erheblich komplexer aufgebaut sein. Die Bereitstellung unterschiedlicher Spannungen und Stromstärken kann schaltungstechnisch auf ganz andere Weise verwirklicht sein. Es können mehr als zwei Schaltstufen zur Einstellung von mehr als zwei einstellbaren Anodenspannungen Uₐ vorgesehen sein. Ebenso können auch die Heizspannung Uₕ und/oder die Stromstärke des Röhrenstroms z.B. mittels des Kathodenschaltnetzes 34i einstellbar sein. Die Schaltnetze 34c, 34i können in der Hochspannungsquelle 34a integriert sein. Im Rahmen dieser Beschreibung kommt es darauf an, dass die Anodenspannung Uₐ mittels des Anodenschaltnetzes 34c einstellbar ist und der Stromfluss durch die Kathode 26b gegebenenfalls mittels des Kathodenschaltnetzes 34i unterbrochen werden kann.

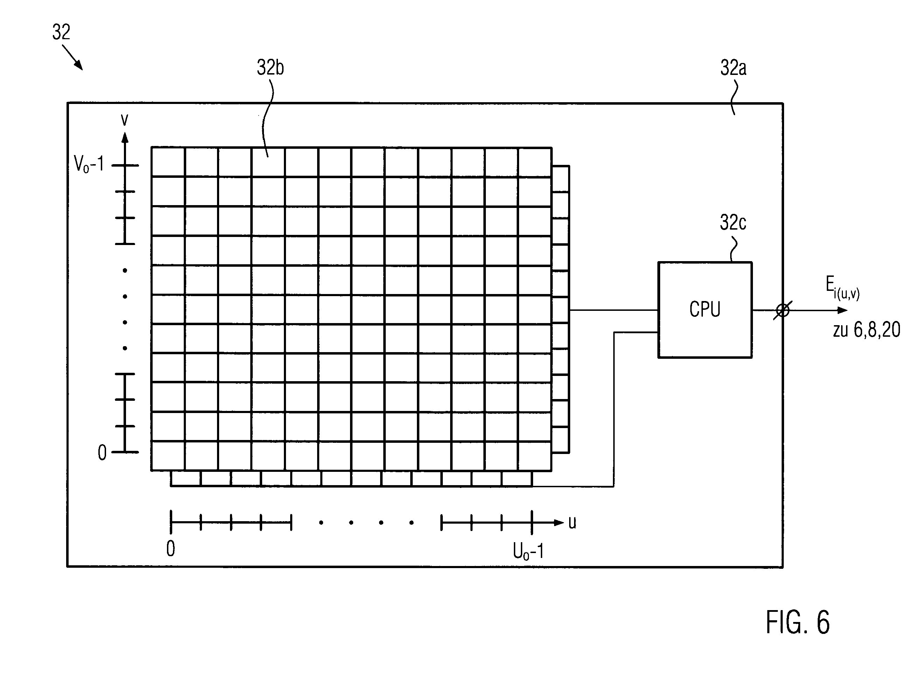

In Fig. 6 ist ein schematischer Aufbau des CCD-Sensors 32 näher dargestellt.

Auf einer Platine 32a sind ein CCD-Chip 32b und eine zentrale Prozessoreinheit (CPU) 32c angeordnet. Der CCD-Chip 32b weist eine in einer zweidimensionalen Matrix angeordnete Anzahl von Fotodioden auf, die im Bereich der Röntgenstrahlung lichtempfindlich sind und ein zur eingestrahlten Lichtmenge (Zahl auftreffender Photonen) proportionales Signal ausgeben. Jede Diode bildet einen Bildpunkt oder Pixel des CCD-Chips 32b und ist durch seine Koordinaten (u,v) charaktisiert. Die Signale der Zeilen und Spalten der Matrix werden von der CPU 32c empfangen und als Einzelbildsignal Eᵢ(u,v) ausgegeben. Der CCD-Sensor ist mit der Bedienkonsole 20 und über das in Fig. 1 skizzierte Netzwerk mit dem Server 6 und/oder der Arbeitsstation 8 verbindbar. Das Einzelbildsignal Eᵢ(u,v) enthält für jedes Pixel (u,v) einen Grauwert, der einer einfallenden Röntgenlichtmenge an diesen Koordinaten des CCD-Chips 32b entspricht.

Zusätzlich kann auf der Platine 32a oder außerhalb davon ein Pufferspeicher vorgesehen sein, um Sensorsignale und/oder Einzelbildsignale zwischenzuspeichern.

Anstelle eines CCD-Sensors 32 kann auch ein anderer digitaler Sensor wie beispielsweise, aber nicht nur, ein CMOS-Sensor mit ausreichender Empfindlichkeit im Röntgenbereich eingesetzt werden.

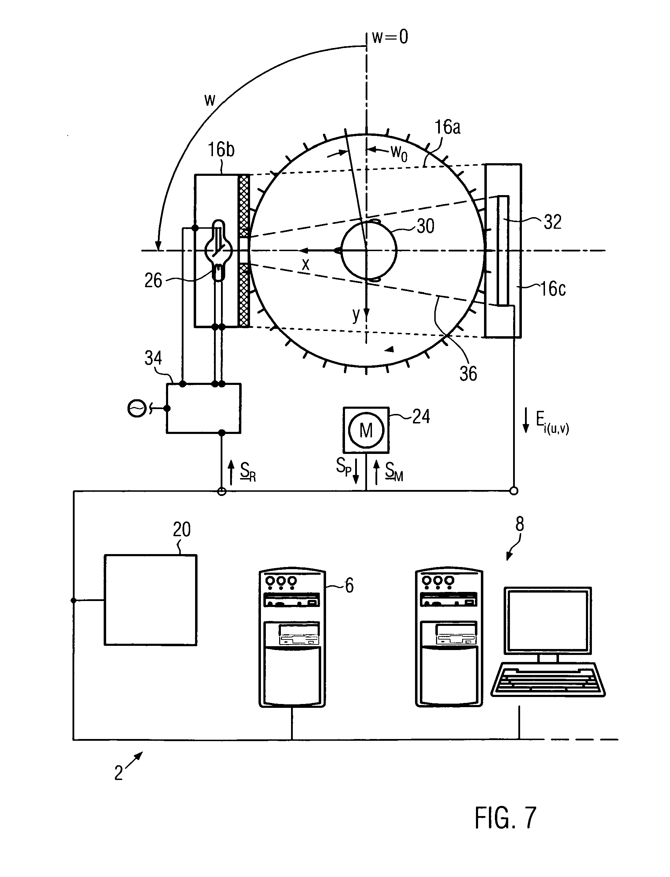

Fig. 7 zeigt noch einmal schematisch die Zusammenschaltung der funktionalen Einheiten des DVT-Systems 2 der vorliegenden Erfindung. Das Diagnoseobjekt 30 ist zwischen dem Belichtungsschenkel 16b mit der Röntgenröhre 26 und dem Sensorschenkel 16c mit dem CCD-Sensor 32 angeordnet. Der Umriss der (oberhalb der Zeichnungsebene liegenden) Basis 16a des C-Arms 16 ist in der Figur durch gepunktete Linien angedeutet. Ein Strahlenkegel 36 von der Röntgenröhre 26 zu dem CCD-Sensor 32 ist durch gestrichelte Linien angedeutet. Obschon das Röntgenvorschaltgerät 34 außerhalb des Belichtungsschenkels 16b dargestellt ist, kann es auch in dem Belichtungsschenkel 16b integriert sein. Der Motor 24 zum Antrieb des C-Arms 16 ist hier isoliert dargestellt, tatsächlich aber mit der Basis 16a des C-Arms 16 verbunden (vgl. Fig. 2).

Die Bedienung des Geräts und die Wahl der Aufnahmeparameter geschieht über die Bedienkonsole 20 und/oder die Arbeitsstation 8. Die Hauptlast der Bildverarbeitung und -speicherung trägt der Server 6. Die Darstellung und Endauswertung der Ergebnisse durch den Benutzer erfolgt an der Arbeitsstation 8, eine Darstellung wenigstens zu Kontrollzwecken kann auch an der Bedienkonsole 20 erfolgen. Die Ansteuerungssignale zur Ansteuerung der Röntgenquelle (s_{R}) und des Motors (s_{M}) können in dem Server 6, der Arbeitsstation 8, der Bedienkonsole 20 oder in einer weiteren, nicht näher dargestellten Verarbeitungseinheit des DVT-Geräts anhand von Vorgabewerten eines Anfangswinkels, eines Endwinkels, eines Winkelinkrements, der zu verwendenden Röntgenfrequenz oder anderer erzeugt werden; die genaue Verteilung der Berechnungs- und Verarbeitungslast kann nach Bedarf und Systemvoraussetzungen festgelegt werden.

In Fig. 7 ist das System in einer Winkellage w=90 ° dargestellt. Ein Winkelschritt oder Winkelinkrement w₀ ist in der Figur aus Gründen der Deutlichkeit mit etwa 10°dargestellt, wird tatsächlich aber kleiner sein (0,1°, 0,25°, 0,5°, 1 °, 2° oder dergleichen).

Wie bereits erläutert, ist das Frequenzspektrum (die Wellenlänge bzw. Energie) der durch die Röntgenröhre 26 erzeugten Röntgenstrahlung von der Höhe der Beschleunigungs- oder Anodenspannung Uₐ abhängig. Daher kann die entstehende Röntgenstrahlung durch die Beschleunigungsspannung Uₐ charakterisiert werden. Die Intensität der Röntgenstrahlung wird durch den Röhrenstrom lₐ bestimmt. Röntgenstrahlung unterschiedlicher Frequenz kann zur Sichtbarmachung unterschiedlicher Gewebearten verwendet werden. Verhältnismäßig niederfrequente Strahlung im Bereich von beispielsweise 40 bis 80 kV ergibt bei weicheren Gewebearten wie Muskeln, Fett, Bindegewebe einen guten Kontrast, während höherfrequente Strahlung im Bereich von beispielsweise 80 bis 120 kV bei dichteren Geweben wie etwa Knochen einen guten Kontrast ergibt.

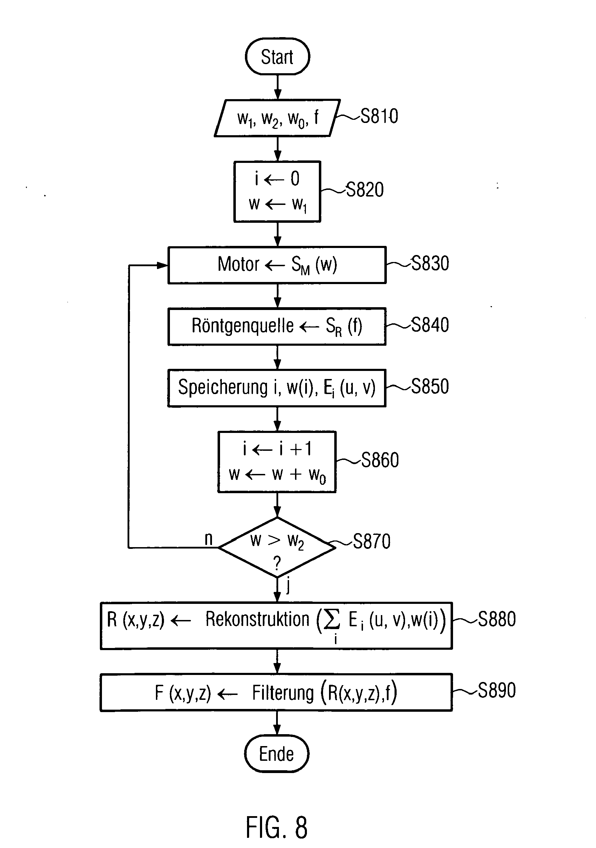

Der Ablauf einer DVT-Aufnahme mit einer gleich bleibenden, vorgebbaren Frequenz (Beschleunigungsspannung Uₐ) ist in einem Ablaufdiagramm in Fig. 8 gezeigt.

Nach dem Beginn der Routine werden in Schritt S810 eine anfängliche Winkellage w₁, eine End-Winkellage w₂, das Winkelinkrement w₀ und ein gewünschtes Frequenzband f eingelesen (etwa durch Eingabe auf der Bedienkonsole 20 oder über die Arbeitsstation 8).

Ein üblicher Winkelbereich reicht z.B. von w₁=0° bis w₂=180° oder w₂=360°; aber auch andere Start- und Endwinkellagen sind möglich. Als Winkelinkrement sind Werte von w₀=0,1 ° bis w₀=2° einstellbar; auch diesbezüglich sind andere Werte möglich.

Anstelle des Frequenzbands f (in PHz oder EHz) kann auch die charakterisierende Anodenspannung Uₐ (in kV) oder die gewünschte Energie (in eV) gewählt werden; damit ist auch das Frequenzband festgelegt. Anstelle des Winkelinkrements w₀ kann auch die Anzahl der gewünschten Einzelbilder gewählt werden; sei n die Anzahl der Einzelbilder, so kann das Winkelinkrement über die Beziehung w₀=(w₂-w₁)/n berechnet werden. Anstelle einer Eingabe der Anfangs-Winkellage w₁ und der End-Winkellage w₂ kann auch ein gewünschter zu überstreichender Winkelbereich gewählt werden sowie der C-Arm 16 manuell in eine gewünschte Anfangslage gedreht und diese Lage als Anfangs-Winkellage w₁ festgelegt werden; sei W der gewünschte Winkelbereich, so kann die End-Winkellage w₂ über die Beziehung w₂=w₁+W berechnet werden.

In dem anschließenden Schritt S820 wird einer Zählvariablen i der Wert 0 zugewiesen und wird dem Sollwert der Winkellage w der Anfangswert w₁ zugewiesen.

In dem anschließenden Schritt S830 wird ein der Winkellage w entsprechendes Motor-Ansteuerungssignal s_{M} berechnet und der Motor mit dem Motor-Ansteuerungssignal s_{M} angesteuert. Hierdurch wird die Winkellage w eingestellt.

In dem anschließenden Schritt S840 wird ein Röntgenquellen-Ansteuerungssignal s_{R} berechnet und die Röntgenquelle mit dem Röntgenquellen-Ansteuerungssignal s_{R} angesteuert. Das Röntgenquellen-Ansteuerungssignal s_{R} veranlasst die Röntgenquelle (Röntgenröhre 26, Röntgenvorschaltgerät 34), das Diagnoseobjekt 30 mit Röntgenstrahlung der vorgegebenen Frequenz f zu beleuchten. Die Beleuchtung ist ein kurzer Impuls, vergleichbar mit einem Röntgenblitz.

In dem anschließenden Schritt S850 werden die Zählvariable i, die zugehörige Winkellage w(i) und das Ausgangssignal (Einzelbildsignal) Eᵢ(u,v) des CCD-Sensors 32 gespeichert.

In dem anschließenden Schritt S860 wird die Zählvariable i um 1 erhöht und wird der Sollwert der Winkellage w um das Winkelinkrement w₀ verändert (erhöht, falls w₂>w₁ bzw. w₀>0).

In dem anschließenden Schritt S870 wird beurteilt, ob der Sollwert der Winkellage w größer als die vorgewählte End-Winkellage w₂ ist oder nicht. Verneinendenfalls springt die Routine zurück zu Schritt S830, wo der Motor 24 auf die neu vorgegebene Winkellage w gefahren wird.

Im Fall einer positiven Beurteilung in Schritt S870 (d.h., wenn die neue Winkellage w größer als die vorgewählte End-Winkellage w₂ ist) verzweigt die Routine zu Schritt S880, wo die Rekonstruktion eines Raumbildes R(x,y,z) anhand der Einzelbilder Eᵢ(u,v) und der zugehörigen Winkellagen w(i) erfolgt.

In dem anschließenden Schritt S890 wird das Raumbild R(x,y,z) einer Filterung unterzogen werden, um ein gefiltertes Raumbild F(x,y,z) zu erhalten. Die Filterung erfolgt anhand einer vorgegebenen Filterungsroutine hinsichtlich Kontrast, Helligkeit und Gamma, die der eingestellten Frequenz angepasst ist.

Danach wird diese Routine beendet.

Die Schritte S820 bis S880 können durch eine Anweisung "Erzeuge Raumbild R(x,y,z) im Frequenzband f" zusammengefasst werden.

In einem alternativen Ablauf können auch die Einzelbilder Eᵢ(u,v) einer Filterung unterzogen werden und die gefilterten Einzelbilder zu einem Raumbild R(x,y,z) rekonstruiert werden. Der Schritt S890 der Filterung des Raumbildes könnte dann entfallen.

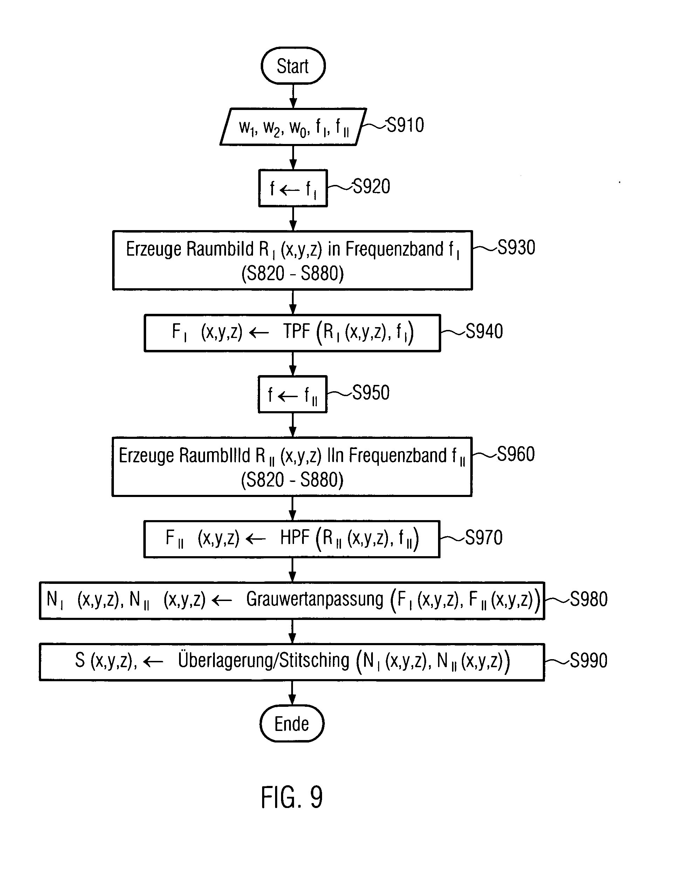

Der Ablauf einer DVT-Aufnahme unter Verwendung zweier vorgebbaren Frequenzen (Beschleunigungsspannungen Uₐ) gemäß einer ersten Ausführungsvariante der vorliegenden Erfindung ist in einem Ablaufdiagramm in Fig. 9 gezeigt.

Nach dem Beginn der Routine werden in Schritt S910 eine anfängliche Winkellage w₁, eine End-Winkellage w₂, das Winkelinkrement w₀ und zwei gewünschte Frequenzbänder f_{I}, f_{II} eingelesen (etwa durch Eingabe auf der Bedienkonsole 20 oder über die Arbeitsstation 8). Dabei wird davon ausgegangen, dass das Frequenzband f_{I} einen niedrigeren Frequenzbereich als das Frequenzband f_{II} abdeckt. Zu Eingabe- und Auswahloptionen sei auf die Anmerkungen zu Schritt S810 verwiesen.

Im anschließenden Schritt S920 wird einem Sollwert der Frequenz f das erste Frequenzband f_{I} zugewiesen.

Im anschließenden Schritt S930 wird ein erstes Raumbild R_{I}(x,y,z) im Frequenzband f_{I} erzeugt und gespeichert. Diese Anweisung entspricht den Schritten S820 bis S880 in Fig. 8.

In dem anschließenden Schritt S940 wird das erste Raumbild R_{I}(x,y,z) einer Filterung mittels Tiefpassfilter (TPF) unterzogen, um ein erstes gefiltertes Raumbild F_{I}(x,y,z) zu erhalten. Die Filterung erfolgt anhand einer vorgegebenen Filterungsroutine, die der eingestellten Frequenz in einem niedrigen Frequenzbereich angepasst ist. Durch die Tiefpassfilterung werden Knochenanteile in dem ersten Raumbild R_{I}(x,y,z) unterdrückt; das erste Raumbild R_{I}(x,y,z) kann daher als "Weichteilbild" bezeichnet werden.

Im anschließenden Schritt S950 wird einem Sollwert der Frequenz f das zweite, höhere Frequenzband f_{II} zugewiesen.

Im anschließenden Schritt S960 wird ein zweites Raumbild R_{II}(x,y,z) im Frequenzband f_{II} erzeugt und gespeichert. Diese Anweisung entspricht den Schritten S820 bis S880 in Fig. 8.

In dem anschließenden Schritt S970 wird das zweite Raumbild R_{I}(x,y,z) einer Filterung mittels Hochpassfilter (HPF) unterzogen, um ein zweites gefiltertes Raumbild F_{II}(x,y,z) zu erhalten. Die Filterung erfolgt anhand einer vorgegebenen Filterungsroutine, die der eingestellten Frequenz in einem höheren Frequenzbereich angepasst ist. Durch die Hochpassfilterung werden Anteile weichen Gewebes in dem zweiten Raumbild R_{II}(x,y,z) unterdrückt; das zweite Raumbild R_{II}(x,y,z) kann daher als "Knochenbild" bezeichnet werden.

In dem anschließenden Schritt S980 erfolgt eine Grauwertanpassung der gefilterten Raumbilder F_{I}(x,y,z), F_{II}(x,y,z), um normalisierte Raumbilder N_{I}(x,y,z), N_{II}(x,y,z) zu erhalten. Geeignete Routinen zur Grauwertanpassung werden später beschrieben.

In dem anschließenden Schritt S990 erfolgt eine Überlagerung der normalisierten Raumbilder N_{I}(x,y,z), N_{II}(x,y,z) zu einem Gesamt-Raumbild S(x,y,z). Die Überlagerung in Schritt S990 umfasst eine Stitching-Routine. Unter Stitching wird ein anatomisch korrektes Zusammenfügen von Bildern derart, dass die zu dem Diagnoseobjekt gehörenden Strukturen in den beiden Raumbildern zusammenpassen, verstanden.

Danach wird diese Routine beendet.

Zusammengefasst wird bei diesem Verfahren das Objekt zwei mal mit unterschiedlichen Röntgenspektren gescannt. Die Bildrekonstruktion in den beiden Röntgenspektren kann grundsätzlich nach bekannten Routinen erfolgen; als Zwischenergebnis werden zwei dreidimensionale Datensätze (Volumenröntgendatensätze) erhalten. Diese werden nach Filterung von Kontrast, Helligkeit und Gamma im Grauwert normalisiert (aneinander angepasst), anatomisch korrekt überlagert und zusammengesetzt.

Der nach dem Zusammensetzen erhaltene Datensatz enthält eine kontrasthöheren Informationsgehalt von Weichteilen, Knochenstrukturen und anderen anatomischen Einzelheiten.

Man beachte, dass die Teilschritte der Rekonstruktion (S880 in S930 und S960) und der Filterung (S940 und S970) zeitaufwändig sein können. Um unnötige Wartezeiten zu vermeiden, ist es denkbar, zunächst alle Einzelbilder in beiden Frequenzbereichen zu erzeugen und zu speichern und erst danach die Rekonstruktion und Filterung für beide Frequenzbereiche vorzunehmen, um dann die Normalisierung der Grauwerte und Überlagerung der Raumbilder (S980 und S990) durchzuführen. Es können auch die Teilschritte zur Rekonstruktion und Filterung hinsichtlich des ersten Frequenzbandes vorgenommen werden, während bereits die Einzelbilder im zweiten Frequenzband aufgenommen werden.

Als eine weitere Abwandlung ist es denkbar, die Einzelbilder im ersten Frequenzband wie vorstehend beschrieben in der Reihenfolge von der Start-Winkellage w₁ zu der End-Winkellage w₂ aufzunehmen und die Einzelbilder im zweiten Frequenzband in umgekehrter Reihenfolge, also von der End-Winkellage w₂ zu der Start-Winkellage w₁, aufzunehmen. Die Drehrichtung des C-Arms 16 bzw. des Motors 24 würde sich also in der End-Winkellage w₂ umkehren.

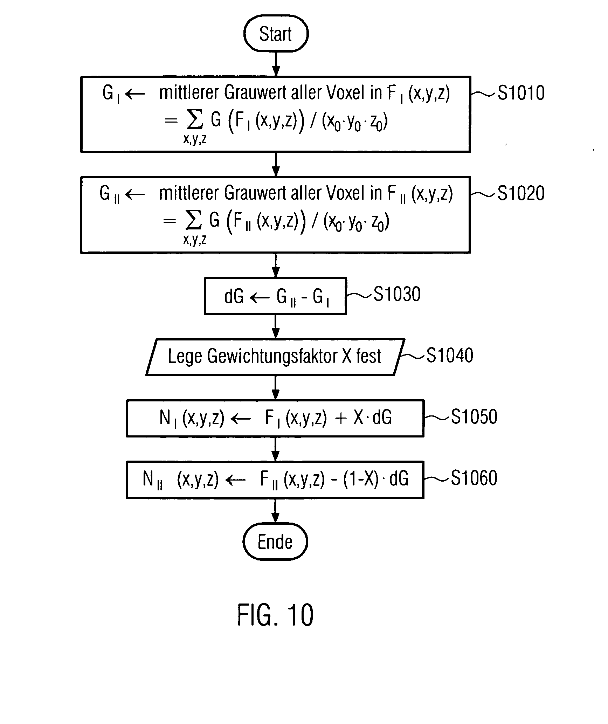

In Fig. 10 ist eine beispielhafte Routine zur Normalisierung der Grauwerte der beiden gefilterten Raumbilder F_{I}(x,y,z), F_{II}(x,y,z) als eine spezielle Ausführungsvariante dargestellt.

Nach Beginn der Routine wird in Schritt S1010 ein erster mittlerer Grauwert G_{I} aus der Summe der Grauwerte der Voxel (Raumbildpunkte) (x,y,z) in dem ersten gefilterten Raumbild F_{I}(x,y,z), geteilt durch die Gesamtzahl X₀.Y₀.Z₀ der Raumpunkte, berechnet.

In dem anschließenden Schritt S1020 wird ein zweiter mittlerer Grauwert G_{II} aus der Summe der Grauwerte der Voxel (x,y,z) in dem zweiten gefilterten Raumbild F_{II}(x,y,z), geteilt durch die Gesamtzahl X₀.Y₀.Z₀ der Raumpunkte, berechnet.

In den Schritten S1010 und S1020 kann die Anzahl der betrachteten Voxel auf diejenigen beschränkt werden, in denen ein Diagnoseobjekt überhaupt identifiziert wurde. Zu diesem Zweck können Schwellwerte definiert werden, die der Grauwert eines Voxels über- oder unterschreiten muss, um als Voxel des Diagnoseobjekts erkannt zu werden.

In dem anschließenden Schritt S1030 wird eine Grauwertdifferenz dG der mittleren Grauwerte der Raumbilder F_{I}(x,y,z), F_{II}(x,y,z) aus der Beziehung dG=G_{II}-G_{I} berechnet.

In dem anschließenden Schritt S1040 wird ein Gewichtungsfaktor X für die Normalisierung der Raumbilder festgelegt. Der Gewichtungsfaktor X ist zwischen 0 und 1 definierbar und gibt an, in welchem Ausmaß das erste gefilterte Raumbild F_{I}(x,y,z) im Grauwert (in der Gesamthelligkeit) dem zweiten gefilterten Raumbild F_{II}(x,y,z) angeglichen werden soll. Der Kehrwert 1-X gibt an, in welchem Ausmaß das zweite gefilterte Raumbild F_{II}(x,y,z) im Grauwert (in der Gesamthelligkeit) dem ersten gefilterten Raumbild F_{I}(x,y,z) angeglichen werden soll. Die Festlegung kann vorgegeben sein, sich aus einer Rechenvorschrift ergeben oder durch einen Benutzer wählbar sein.

In dem anschließenden Schritt S1050 wird das erste gefilterte Raumbild F_{I}(x,y,z) anhand der in Schritt S1030 berechneten Grauwertdifferenz dG und dem in Schritt S1040 festgelegten Gewichtungsfaktor X normalisiert. Hierzu wird jedem Voxel des ersten gefilterten Raumbildes F_{I}(x,y,z) die mit dem Gewichtungsfaktor X gewichtete Grauwertdifferenz dG hinzuaddiert, um nach der Beziehung N_{I}(x,y,z)=F_{I}(x,y,z)+X.dG das erste normalisierte Raumbild N_{I}(x,y,z) zu erhalten.

In dem anschließenden Schritt S1060 wird das zweite gefilterte Raumbild F_{II}(x,y,z) anhand der in Schritt S1030 berechneten Grauwertdifferenz dG und dem in Schritt S1040 festgelegten Gewichtungsfaktor X normalisiert. Hierzu wird jedem Voxel des zweiten gefilterten Raumbildes F_{II}(x,y,z) die mit dem Kehrwert (1-X) des Gewichtungsfaktors X gewichtete Grauwertdifferenz dG hinzuaddiert, um nach der Beziehung N_{II}(x,y,z)=F_{II}(x,y,z)+(1-X).dG das zweite normalisierte Raumbild N_{II}(x,y,z) zu erhalten.

Danach wird diese Routine beendet.

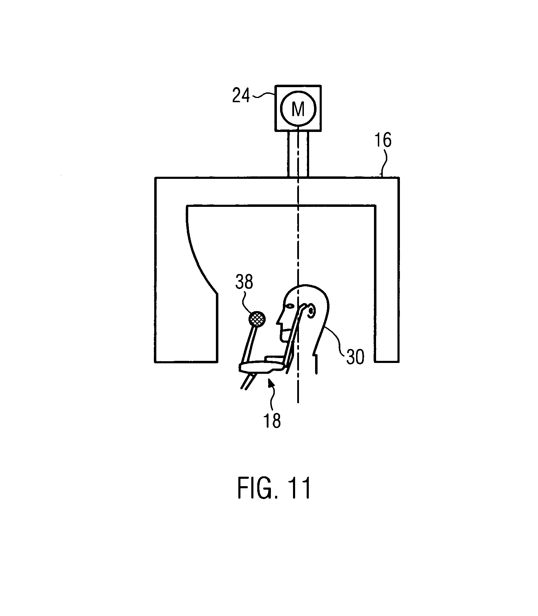

In Fig. 11 ist eine weitere Ausführungsvariante der vorliegenden Erfindung angedeutet. Dabei befindet sich der Kopf des Patienten als Diagnoseobjekt 30 zwischen den Schenkeln des durch den Motor 24 angetriebenen C-Arms 16 und ruht auf der Kinnauflage des Positionierungstisches 18. An dem Positionierungstisch 18 ist ferner ein Referenzkörper 38 fest angebracht. Der Referenzkörper 38 weist hier die Form einer Kugel auf. Die Form des Referenzkörpers 38 ist jedoch frei wählbar; es kann also insbesondere auch ein Würfel, Quader, eine Pyramide, ein Zylinder oder ein anderer Körper sein.

Der Referenzkörper 38 wird bei jeder Aufnahme eines Einzelbildes Eᵢ(u,v) mit abgebildet, jedes Mal aus einem anderen Winkel. Die Form und die Raumposition des Referenzkörpers 38 können durch eine Bildverarbeitung in den resultierenden Raumbildern R_{I}(x,y,z), R_{II}(x,y,z) bzw. den gefilterten Raumbildern F_{I}(x,y,z), F_{II}(x,y,z) leicht erkannt werden. Diese Information kann zur Normierung der Grauwerte vorteilhaft ausgenutzt werden.

Der Referenzkörper 38 ist vorzugsweise aus einem Kunststoff hergestellt und weist eine Dotierung mit einem Röntgenstrahlen absorbierenden Material (ein Röntgenkontrastmaterial) auf. Die Materialien und die Dotierungsdichte des Referenzobjekts 38 sind so gewählt, dass seine Absorptions-, Reflexions- und Transmissionseigenschaften für Röntgenstrahlen denjenigen des Diagnoseobjekts 30 ähnlich sind, dass insbesondere die Grauwertunterschiede in einem Einzelbild Eᵢ(u,v) bei Bestrahlung mit unterschiedlichen Röntgenspektren denjenigen des Diagnoseobjekts 30 ähnlich sind. Das Dotierungsmaterial kann beispielsweise Bariumsulfat, Eisen, Titan oder dergleichen enthalten. Dabei können die besonderen Eigenschaften, wenn das Dotierungsmaterial als Nanopartikel vorliegt, ausgenutzt werden.

Es ist anzumerken, dass als Referenzobjekt jedes Objekt verwendet werden kann, das in beiden Röntgenfrequenzbereichen deutlich abgebildet wird. Grundsätzlich kann es sich auch um einen Teil des Diagnoseobjekts selbst handeln, das mittels einer Bilderkennungssoftware automatisch erkannt wird.

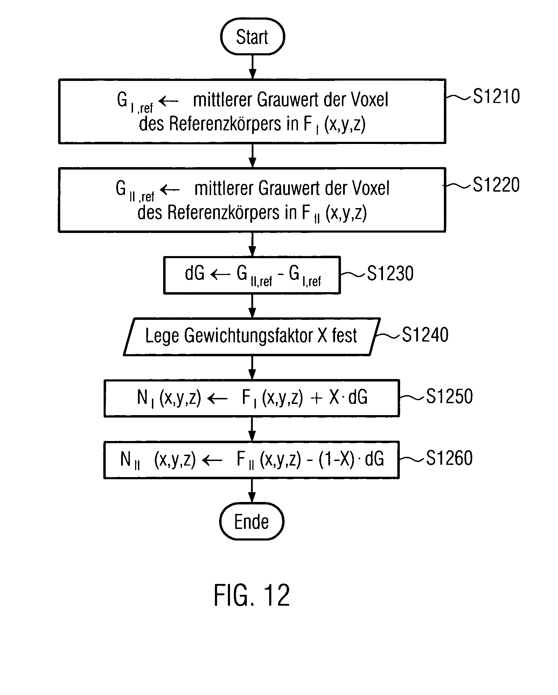

Fig. 12 zeigt eine beispielhafte Routine zur Normalisierung der Grauwerte der beiden gefilterten Raumbilder F_{I}(x,y,z), F_{II}(x,y,z) in dieser Ausführungsvariante.

Nach Beginn der Routine wird in Schritt S1210 ein mittlerer Grauwert G_{I,ref} der dem Referenzkörper 38 zuordenbaren Voxel des ersten gefilterten Raumbildes F_{I}(x,y,z) ermittelt.

In dem anschließenden Schritt S1220 wird ein mittlerer Grauwert G_{II,ref} der dem Referenzkörper 38 zuordenbaren Voxel des zweiten gefilterten Raumbildes F_{II}(x,y,z) ermittelt.

In dem anschließenden Schritt S1230 wird eine Grauwertdifferenz dG als Differenz der in den Schritten S1210 und S1220 mittleren Grauwerte G_{I,ref} und G_{II,ref} nach der Beziehung dG=G_{II,ref}-G_{I,ref} ermittelt.

In dem anschließenden Schritt S1240 wird ein Gewichtungsfaktor X für die Normalisierung der Raumbilder festgelegt. Der Gewichtungsfaktor X ist zwischen 0 und 1 definierbar und gibt an, in welchem Ausmaß das erste gefilterte Raumbild F_{I}(x,y,z) im Grauwert (in der Gesamthelligkeit) dem zweiten gefilterten Raumbild F_{II}(x,y,z) angeglichen werden soll. Der Kehrwert 1-X gibt an, in welchem Ausmaß das zweite gefilterte Raumbild F_{II}(x,y,z) im Grauwert (in der Gesamthelligkeit) dem ersten gefilterten Raumbild F_{I}(x,y,z) angeglichen werden soll. Die Festlegung kann vorgegeben sein, sich aus einer Rechenvorschrift ergeben oder durch einen Benutzer wählbar sein.

In dem anschließenden Schritt S1250 wird das erste gefilterte Raumbild F_{I}(x,y,z) anhand der in Schritt S1230 berechneten Grauwertdifferenz dG und dem in Schritt S1240 festgelegten Gewichtungsfaktor X normalisiert. Hierzu wird jedem Voxel des ersten gefilterten Raumbildes F_{I}(x,y,z) die mit dem Gewichtungsfaktor X gewichtete Grauwertdifferenz dG hinzuaddiert, um nach der Beziehung N_{I}(x,y,z)=F_{I}(x,y,z)+X.dG das erste normalisierte Raumbild N_{I}(x,y,z) zu erhalten.

In dem anschließenden Schritt S1260 wird das zweite gefilterte Raumbild F_{II}(x,y,z) anhand der in Schritt S1230 berechneten Grauwertdifferenz dG und dem in Schritt S1240 festgelegten Gewichtungsfaktor X normalisiert. Hierzu wird jedem Voxel des zweiten gefilterten Raumbildes F_{II}(x,y,z) die mit dem Kehrwert (1-X) des Gewichtungsfaktors X gewichtete Grauwertdifferenz dG hinzuaddiert, um nach der Beziehung N_{II}(x,y,z)=F_{II}(x,y,z)+(1-X).dG das zweite normalisierte Raumbild N_{II}(x,y,z) zu erhalten.

Danach wird diese Routine beendet.

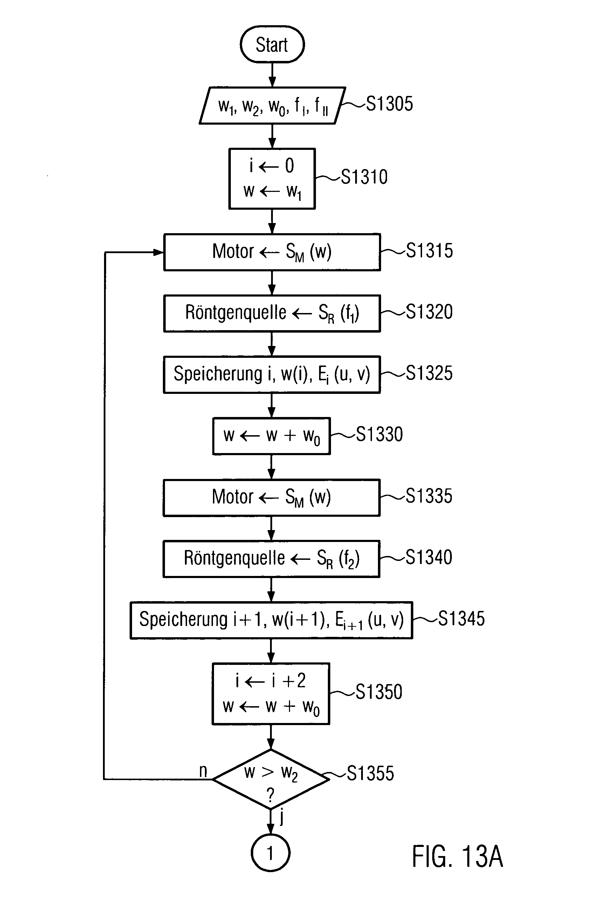

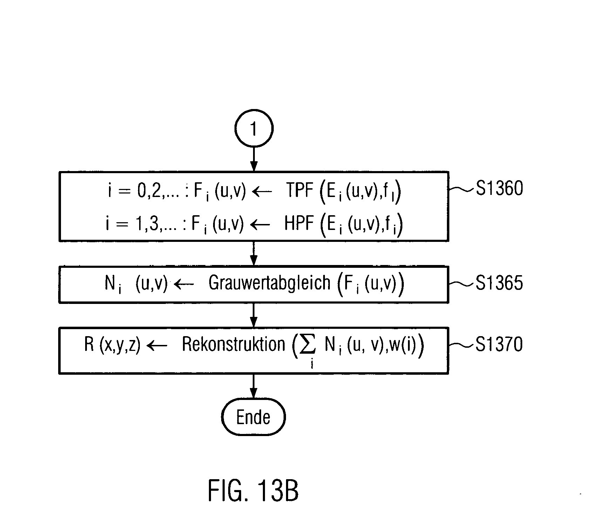

Der Ablauf einer DVT-Aufnahme unter Verwendung zweier vorgebbaren Frequenzen (Beschleunigungsspannungen Uₐ) gemäß einer weiteren grundsätzlichen Ausführungsvariante der vorliegenden Erfindung ist in einem Ablaufdiagramm in Fig. 13A und 13B gezeigt.

Nach dem Beginn der Routine werden in Schritt S1305 eine anfängliche Winkellage w₁, eine End-Winkellage w₂, ein Winkelinkrement w₀ und zwei gewünschte Frequenzbänder f_{I}, f_{II} eingelesen (etwa durch Eingabe auf der Bedienkonsole 20 oder über die Arbeitsstation 8). Zu Eingabe- und Auswahloptionen sei auf die Anmerkungen zu Schritt S81 0 verwiesen.

In dem anschließenden Schritt S1310 wird einer Zählvariablen i der Wert 0 zugewiesen und wird dem Sollwert der Winkellage w der Anfangswert w₁ zugewiesen.

In dem anschließenden Schritt S1315 wird ein der Winkellage w entsprechendes Motor-Ansteuerungssignal s_{M} berechnet und der Motor mit dem Motor-Ansteuerungssignal s_{M} angesteuert. Hierdurch wird die Winkellage w eingestellt.

In dem anschließenden Schritt S1320 wird ein Röntgenquellen-Ansteuerungssignal s_{R}(f_{I}) berechnet und die Röntgenquelle mit dem Röntgenquellen-Ansteuerungssignal s_{R}(f_{I}) angesteuert. Das Röntgenquellen-Ansteuerungssignal s_{R} ist so festgelegt, dass es die Röntgenquelle (Röntgenröhre 26, Röntgenvorschaltgerät 34) veranlasst, das Diagnoseobjekt 30 mit Röntgenstrahlung mit einem Röntgenimpuls der ersten vorgegebenen Frequenz f_{I} zu beleuchten.

In dem anschließenden Schritt S1325 werden die Zählvariable i, die zugehörige Winkellage w(i) und das Ausgangssignal (Einzelbildsignal) Eᵢ(u,v) des CCD-Sensors 32 gespeichert.

In dem anschließenden Schritt S1330 wird wird der Sollwert der Winkellage w um das Winkelinkrement w₀ verändert (erhöht, falls w₂>w₁ bzw. w₀>0).

In dem anschließenden Schritt S1335 wird ein der neuen Winkellage w entsprechendes Motor-Ansteuerungssignal s_{M} berechnet und der Motor mit dem Motor-Ansteuerungssignal s_{M} angesteuert. Hierdurch die Winkellage w eingestellt.

In dem anschließenden Schritt S1340 wird ein Röntgenquellen-Ansteuerungssignal s_{R}(f_{I}) berechnet und die Röntgenquelle mit dem Röntgenquellen-Ansteuerungssignal s_{R}(f_{II}) angesteuert. Das Röntgenquellen-Ansteuerungssignal s_{R} ist so festgelegt, dass es die Röntgenquelle (Röntgenröhre 26, Röntgenvorschaltgerät 34) veranlasst, das Diagnoseobjekt 30 mit Röntgenstrahlung mit einem Röntgenimpuls der ersten vorgegebenen Frequenz f_{II} zu beleuchten.

In dem anschließenden Schritt S1345 werden der um 1 erhöhte Wert i+1 der Zählvariable i, die zugehörige Winkellage w(i+1) und das Ausgangssignal (Einzelbildsignal) Eᵢ₊₁(u,v) des CCD-Sensors 32 gespeichert.

In dem anschließenden Schritt S1350 wird die Zählvariable i um 2 erhöht und wird der Sollwert der Winkellage w um das Winkelinkrement w₀ verändert (erhöht, falls w₂>w₁ bzw. w₀>0).

In dem anschließenden Schritt S1355 wird beurteilt, ob der Sollwert der Winkellage w größer als die vorgegebene End-Winkellage w₂ ist oder nicht. Verneinendenfalls wird springt die Routine zurück zu Schritt S1315, wo der Motor 24 auf die neu vorgegebene Winkellage w gefahren wird.

Im Fall einer positiven Beurteilung in Schritt S1350 (d.h., wenn die neue Winkellage w größer oder gleich der vorgewählte End-Winkellage w₂ ist) verzweigt die Routine zu Schritt S1360 (Verzweigungs- bzw. Übergabestelle in in Fign. 13A und 13B). In diesem Schritt S1360 werden die Einzelbilder Eᵢ(u,v) einer Filterung unterzogen, um gefilterte Einzelbilder Fᵢ(u,v) zu erhalten. Die Filterung erfolgt anhand einer vorgegebenen Filterungsroutine hinsichtlich Kontrast, Helligkeit und/oder Gamma, die der eingestellten Frequenz angepasst ist. Und zwar werden die Einzelbilder Eᵢ(u,v) mit geraden Werten von i, also diejenigen Einzelbilder Eᵢ(u,v), die in dem ersten Frequenzband f_{I} aufgenommen wurden, einer Tiefpassfilterung unterzogen, und werden die Einzelbilder Eᵢ(u,v) mit ungeraden Werten von i, also diejenigen Einzelbilder Eᵢ(u,v), die in dem zweiten, höheren Frequenzband f_{II} aufgenommen wurden, einer Hochpassfilterung unterzogen.

In dem anschließenden Schritt S1365 werden die gefilterten Einzelbilder Fᵢ(u,v) einem Grauwertabgleich unterzogen, um normalisierte Einzelbilder Nᵢ(u,v) zu erhalten.

In dem anschließenden Schritt S1370 wird eine dreidimensionale Darstellung, also ein Raumbild R(x,y,z) aus den normalisierten Einzelbildern Nᵢ(u,v) und den jeweils zugehörigen Winkellagen w(i) rekonstruiert.

Danach wird diese Routine beendet.

In einem alternativen Ablauf kann die Filterung der Einzelbilder Eᵢ(u,v) bereits während der Abtastungssequenz der Schritte S1310 bis S1355 jeweils nach der Speicherung der Einzelbilder Eᵢ(u,v) erfolgen (oder es werden nur die gefilterten Einzelbilder gespeichert). Der Schritt S1360 der Filterung Einzelbilder nach der Abtastungssequenz würde dann entfallen.

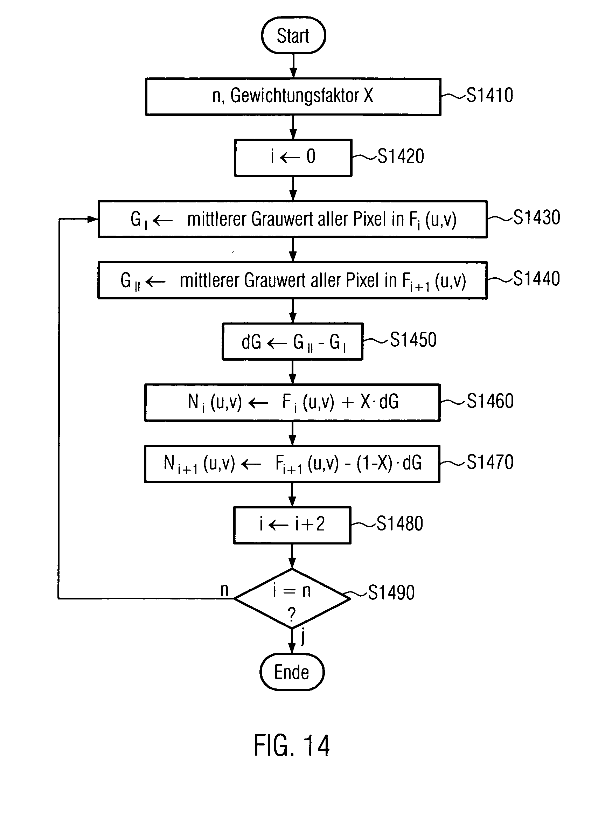

In Fig. 14 ist eine beispielhafte Routine zur Normalisierung der Grauwerte der gefilterten Einzelbilder Fᵢ(u,v) als eine spezielle Ausführungsvariante dargestellt.

Nach Beginn der Routine wird in S1410 die Gesamtzahl n der gefilterten Einzelbilder Fᵢ(u,v) sowie ein Gewichtungsfaktor X für die Normalisierung der Raumbilder initialisiert. Die Gesamtzahl n der der gefilterten Einzelbilder Fᵢ(u,v) wird beispielsweise aus einem Speicher eingelesen. Der Gewichtungsfaktor X ist zwischen 0 und 1 definierbar und gibt an, in welchem Ausmaß die in dem ersten Frequenzband aufgenommenen Einzelbilder im Grauwert (in der Gesamthelligkeit) den in dem zweiten Frequenzband aufgenommenen Einzelbildern angeglichen werden sollen. Der Kehrwert 1-X gibt an, in welchem Ausmaß die in dem zweiten Frequenzband aufgenommenen Einzelbilder im Grauwert (in der Gesamthelligkeit) den in dem ersten Frequenzband aufgenommenen Einzelbildern angeglichen werden sollen. Der Gewichtungsfaktor X kann fest voreingestellt sein, von einem Benutzer über eine Benutzerschnittstelle, z.B. die Arbeitsstation 8, wählbar sein oder über eine Rechenvorschrift anhand der Frequenzbänder, in welchen die Einzelbilder Eᵢ(u,v) in der Routine von Fig. 13A, 13B aufgenommen worden sind, ermittelt werden.

In dem anschließenden Schritt S1420 wird eine Zählvariable i auf Null gesetzt.

In dem anschließenden Schritt S1430 ein erster mittlerer Grauwert G_{I} aus der Summe der Grauwerte der Pixel (Bildpunkte) (u,v) in dem gefilterten Einzelbild Fᵢ(u,v), geteilt durch die Anzahl der Bildpunkte, berechnet.

In dem anschließenden Schritt S1440 ein erster mittlerer Grauwert G_{II} aus der Summe der Grauwerte der Pixel (Bildpunkte) (u,v) in dem gefilterten Einzelbild Fᵢ₊₁(u,v), geteilt durch die Anzahl der Bildpunkte, berechnet.

In den Schritten S1430 und S1440 kann die Anzahl der betrachteten Pixel auf diejenigen beschränkt werden, in denen ein Diagnoseobjekt überhaupt identifiziert wurde. Zu diesem Zweck kann eine Rauschschwelle definiert werden, die der Grauwert eines Pixels über- oder unterschreiten muss, um als Pixel des Diagnoseobjekts erkannt zu werden.

In dem anschließenden Schritt S1450 wird eine Grauwertdifferenz dG der mittleren Grauwerte der benachbarten Raumbilder Fᵢ(u,v), Fᵢ₊₁(u,v) aus der Beziehung dG=G_{II}-G_{I} berechnet.

In dem anschließenden Schritt S1460 wird das gefilterte Einzelbild Fᵢ(u,v) anhand der in Schritt S1420 berechneten Grauwertdifferenz dG und dem in Schritt S1410 festgelegten Gewichtungsfaktor X normalisiert. Hierzu wird jedem Pixel des gefilterten Einzelbildes Fᵢ(u,v) die mit dem Gewichtungsfaktor X gewichtete Grauwertdifferenz dG hinzuaddiert, um nach der Beziehung Nᵢ(u,v)=Fᵢ(u,v)+X.dG ein normalisiertes Einzelbild Nᵢ(u,v) zu erhalten.

In dem anschließenden Schritt S1470 wird das benachbarte gefilterte Einzelbild Fᵢ₊₁(u,v) anhand der in Schritt S1420 berechneten Grauwertdifferenz dG und dem in Schritt S1410 festgelegten Gewichtungsfaktor X normalisiert. Hierzu wird jedem Pixel des gefilterten Einzelbildes Fᵢ₊₁(u,v) die mit dem Kehrwert (1-X) des Gewichtungsfaktors X gewichtete Grauwertdifferenz dG hinzuaddiert, um nach der Beziehung Nᵢ₊₁(u,v)=Fᵢ₊₁(u,v)+(1-X).dG ein normalisiertes Einzelbild Nᵢ₊₁(u,v) zu erhalten.

In dem anschließenden Schritt S1480 wird die Zählvariable i um den Wert 2 erhöht.

In dem anschließenden Schritt S1490 wird beurteilt, ob die Zählvariable i die Gesamtzahl n der Einzelbilder erreicht hat. Verneinendenfalls springt die Routine zurück zu Schritt S1430, und der Grauwertabgleich wird für das nächste Paar von Einzelbildern durchgeführt.

Im Fall einer positiven Beurteilung in Schritt S1490 (d.h., wenn die Anzahl der Einzelbilder erreicht ist) wird diese Routine beendet.

In einer Abwandlung kann der Grauwertabgleich auch während der Abtastung (Schritte 1310 bis 1355 in Fig. 13A) durchgeführt werden. Dazu werden die Schritte 1430 bis 1470 von Fig. 14 nach der Speicherung des zweiten Einzelbildes eines Einzelbildpaares (Schritt 1345 in Fig. 13A) durchgeführt. Diese Abwandlung erfordert, dass vor dem Grauwertabgleich die Filterung der Einzelbilder dieses Einzelbildpaar durchgeführt wird.

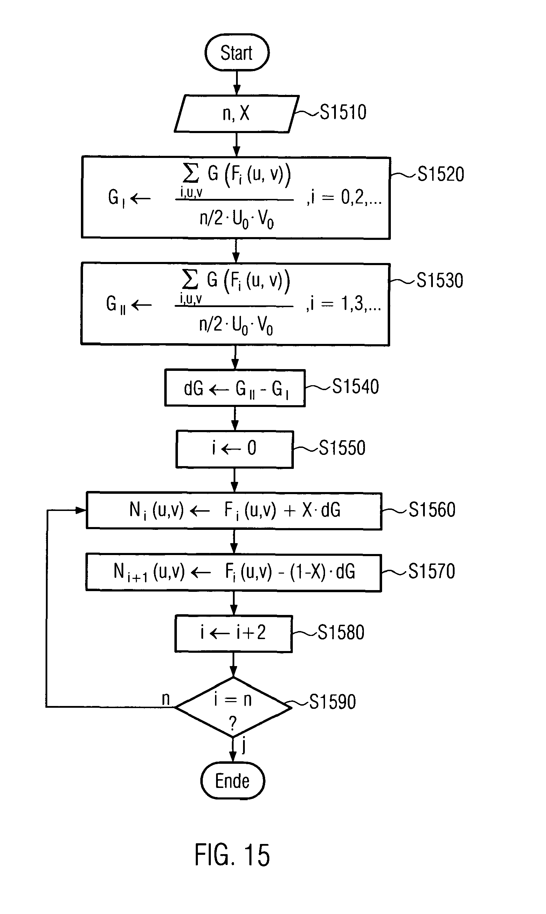

In Fig. 15 ist eine alternative Routine zur Normalisierung der Grauwerte der gefilterten Einzelbilder Fᵢ(u,v) dargestellt.

Nach Beginn der Routine wird in S1510 die Gesamtzahl n der gefilterten Einzelbilder Fᵢ(u,v) sowie ein Gewichtungsfaktor X für die Normalisierung der Raumbilder initialisiert. Zur Initialisierung der Parameter n und X wird auf die Ausführungen zu Schritt S1410 Bezug genommen.

In dem anschließenden Schritt S1520 ein erster mittlerer Grauwert G_{I} aus der Summe der Grauwerte der Pixel (Bildpunkte) (u,v) aller gefilterten Einzelbilder Fᵢ(u,v) mit geradzahligem i, geteilt durch die Gesamtzahl der Bildpunkte U₀.V₀ und den halben Wert n/2 der Gesamtzahl der Bilder, berechnet.

In dem anschließenden Schritt S1530 ein erster mittlerer Grauwert G_{II} aus der Summe der Grauwerte der Pixel (Bildpunkte) (u,v) aller gefilterten Einzelbilder Fᵢ(u,v) mit ungeradzahligem i, geteilt durch die Anzahl der Bildpunkte U₀.V₀ und den halben Wert n/2 der Gesamtzahl der Bilder, berechnet.

In den Schritten S1520 und S1530 kann die Anzahl der betrachteten Pixel auf diejenigen beschränkt werden, in denen ein Diagnoseobjekt überhaupt identifiziert wurde. Zu diesem Zweck kann eine Rauschschwelle definiert werden, die der Grauwert eines Pixels über- oder unterschreiten muss, um als Pixel des Diagnoseobjekts erkannt zu werden.

In dem anschließenden Schritt S1540 wird eine Grauwertdifferenz dG der mittleren Grauwerte der Raumbilder Fᵢ(u,v) aus der Beziehung dG=G_{II}-G_{I} berechnet.

In dem anschließenden Schritt S1550 wird eine Zählvariable i auf Null gesetzt.

In dem anschließenden Schritt S1560 wird das gefilterte Einzelbild Fᵢ(u,v) anhand der in Schritt S1550 berechneten Grauwertdifferenz dG und dem in Schritt S1510 festgelegten Gewichtungsfaktor X normalisiert. Hierzu wird jedem Pixel des gefilterten Einzelbildes Fᵢ(u,v) die mit dem Gewichtungsfaktor X gewichtete Grauwertdifferenz dG hinzuaddiert, um nach der Beziehung Nᵢ(u,v)=Fᵢ(u,v)+X.dG ein normalisiertes Einzelbild Nᵢ(u,v) zu erhalten.

In dem anschließenden Schritt S1570 wird das benachbarte gefilterte Einzelbild Fᵢ₊₁(u,v) anhand der in Schritt S1550 berechneten Grauwertdifferenz dG und dem in Schritt S1510 festgelegten Gewichtungsfaktor X normalisiert. Hierzu wird jedem Pixel des gefilterten Einzelbildes Fᵢ₊₁(u,v) die mit dem Kehrwert (1-X) des Gewichtungsfaktors X gewichtete Grauwertdifferenz dG hinzuaddiert, um nach der Beziehung Nᵢ₊₁(u,v)=Fᵢ₊₁(u,v)+(1-X).dG ein normalisiertes Einzelbild Nᵢ₊₁(u,v) zu erhalten.

In dem anschließenden Schritt S1580 wird die Zählvariable i um den Wert 2 erhöht.

In dem anschließenden Schritt S1590 wird beurteilt, ob die Zählvariable i die Gesamtzahl n der Einzelbilder erreicht hat. Verneinendenfalls springt die Routine zurück zu Schritt S1560, und der Grauwertabgleich wird für das nächste Paar von Einzelbildern durchgeführt.

Im Fall einer positiven Beurteilung in Schritt S1590 (d.h., wenn die Anzahl der Einzelbilder erreicht ist) wird diese Routine beendet.

Der Grauwertabgleich der gefilterten Einzelbilder Fᵢ(u,v) kann auch anhand eines Referenzkörpers gemäß der Anordnung in Fig. 11 durchgeführt werden, wenn in den Routinen gemäß Fig. 14 oder 15 anstelle eines mittleren Grauwerts aller Pixel der Einzelbilder der mittlere Grauwert der Pixel des Referenzkörpers in den Einzelbildern verwendet wird, um die Grauwertdifferenz zu ermitteln. Die Identifizierung des Referenzkörpers in den Einzelbildern ist anhand einer Bilderkennungsroutine leicht möglich.

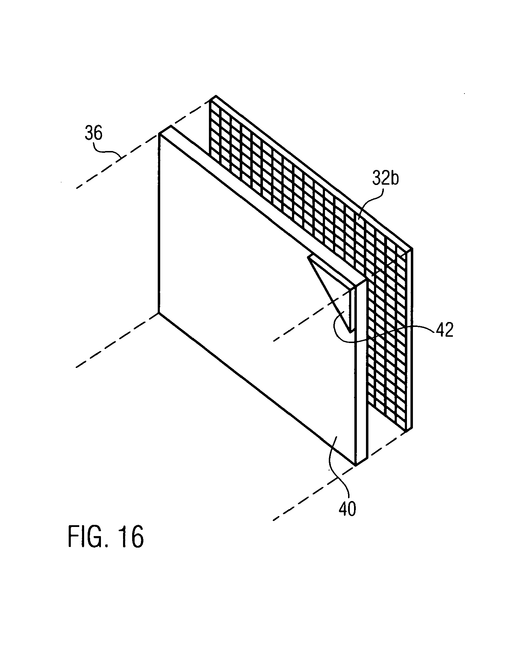

Fig. 16 zeigt als eine Abwandlung die Anordnung eines CCD-Chips mit einem anderen Referenzkörper in einer perspektivischen Explosionsansicht als eine weitere Ausführungsvariante der vorliegenden Erfindung.

Vor dem CCD-Chip 32b (zum Aufbau des CCD-Sensors 32 insgesamt wird ergänzend auf Fig. 6 und zugehörige Beschreibung Bezug genommen) befindet sich eine Schutzglasscheibe 40 im Strahlengang (Strahlenkegel) 36 der Röntgenquelle. In einer Ecke der Schutzglasscheibe ist ein Referenzkörper 42 im Strahlengang 36 angebracht. Der Referenzkörper 42 ist in dieser Abwandlung ein Folienstück dreieckiger Form; die Form des Referenzkörpers 42 ist jedoch frei wählbar. Der Referenzkörper 42 ist vorteilhafterweise aus einer Kunststoff-Folie hergestellt und weist eine Beschichtung mit einem Röntgenstrahlen absorbierenden Material (ein Röntgenkontrastmaterial) auf. Die Materialien und die Beschichtungsdichte des Referenzobjekts 42 sind so gewählt, dass seine Absorptions-, Reflexions- und Transmissionseigenschaften für Röntgenstrahlen denjenigen des Diagnoseobjekts (30, hier nicht abgebildet) ähnlich sind, dass insbesondere die Grauwertunterschiede in einem Einzelbild Eᵢ(u,v) bei Bestrahlung mit unterschiedlichen Röntgenspektren ähnlich demjenigen des Diagnoseobjekts (30) ist. Als Beschichtungsmaterialien kann auf die im Zusammenhang mit der Ausführungsvariante des Referenzkörpers 38 in Fig. 11 zurückgegriffen werden. Anstelle einer Beschichtung ist auch hier eine Dotierung möglich.

Der Referenzkörper 42 wird bei jeder Aufnahme eines Einzelbildes Eᵢ(u,v) an der gleichen Stelle der zweidimensionalen Einzelbildmatrix mit abgebildet. Die Erkennung des Referenzkörpers 42 ist daher besonders einfach.

In der vorstehenden Beschreibung wurde davon ausgegangen, dass eine Röntgenröhre (26, vgl. Fig. 5) unter Ansteuerung verschiedener Beschleunigungsspannungen Uₐ (und/oder Röhrenströme Iₐ und/oder Heizspannungen Uₕ) durch ein Röntgenvorschaltgerät (34, vgl. Fig. 5) Röntgenspektren oder -frequenzbänder in unterschiedlichen Frequenzbereichen aussenden kann. Hierzu sind, insbesondere in den Varianten mit einer Umschaltung des Frequenzbereichs nach jedem Winkelinkrement, Röntgenröhren und -vorschaltgeräte mit möglichst schneller Reaktionszeit, steilen Anstiegsflanken und präziser Steuerbarkeit bereitzustellen. Die apparatetechnischen Anforderungen können daher hoch sein. Die Innenkapazität der Röntgenröhre sollte so gering wie möglich sein; dies kann z.B. einen möglichst kleinen Wehnelt-Zylinder erfordern. Zur Umschaltung der Spannung kann eine ultraschnelle Schaltkaskade ähnlich den Hochspannungszündungen in einem Kraftfahrzeug vorgesehen sein.

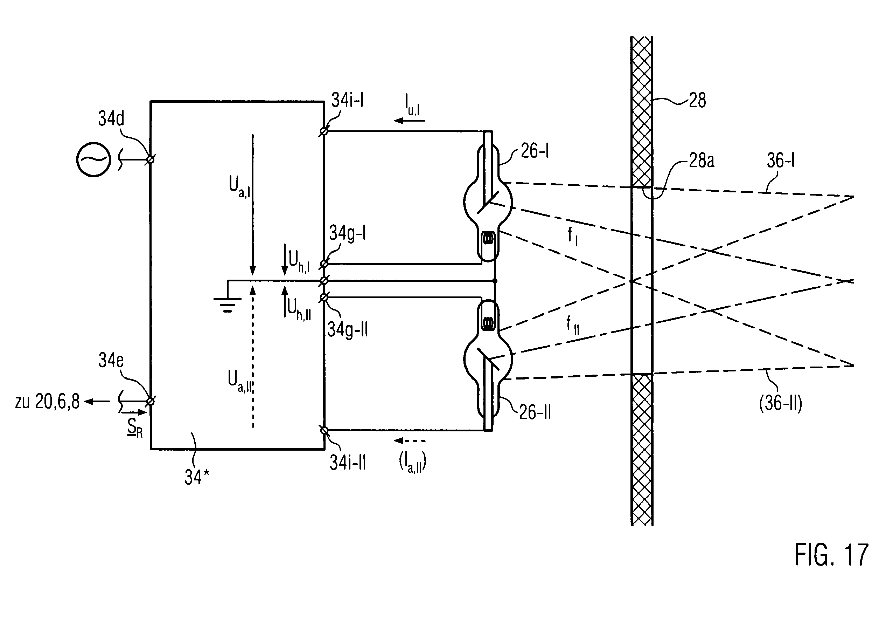

Fig. 17 zeigt schematisch eine Ausführungsvariante mit zwei Röntgenröhren 26-I, 26-II, die von einem gemeinsamen Röntgenvorschaltgerät 34* angesteuert werden. Zum Verständnis der Figur soll angenommen werden, dass die Röntgenröhre 26-I aktiv ist, während die zweite Röntgenröhre 26-II nicht aktiv ist. Das ist in der Figur so angedeutet, dass Vorgänge und Zustände, die mit der zweiten Röntgenröhre 26-II zu tun haben, gegenüber Vorgängen und Zuständen, die mit der ersten Röntgenröhre 26-I zu tun haben, in abgeschwächter Form (dünnere Linien, gestrichtelte anstelle durchgezogenener Linien, Bezugszeichen in Klammern) dargestellt sind.

Das Röntgenvorschaltgerät 34* ist ähnlich wie das Röntgenvorschaltgerät 34 in der zuvor anhand der Fig. 5 beschriebenen Ausführungsvariante, doch kann das Röntgenvorschaltgerät 34* der vorliegenden Ausführungsvariante wahlweise zwei Beschleunigungsspannungen und zwei Heizspannungen getrennt voneinander bereitstellen. An einem Netzanschluss 34d ist eine Netzspannung anschließbar. An einem Signaleingang 34e ist ein Röntgen-Ansteuerungssignal s_{R} zuführbar. An einem ersten Anodenspannungsausgang 34i-I ist eine erste Anodenspannung (Beschleunigungsspannung) U_{a,I} bereitstellbar. An einem ersten Kathodenspannungsausgang 34g-I ist eine erste Kathodenspannung (Heizspannung) U_{h,I} abgreifbar. An einem zweiten Anodenspannungsausgang 34i-II ist eine zweite Anodenspannung (Beschleunigungsspannung) U_{a,II} bereitstellbar. An einem zweiten Kathodenspannungsausgang 34g-II ist eine zweite Kathodenspannung (Heizspannung) U_{h,II} abgreifbar. Ein Masseausgang (nicht näher bezeichnet) stellt ein gemeinsames Massepotential bereit.

Die erste Röntgenröhre 26-I ist mit dem ersten Anodenspannungsausgang 34i-I, dem ersten Kathodenspannungsausgang 34g-I und dem Masseanschluss des Röntgenvorschaltgeräts 34* verbunden. Die zweite Röntgenröhre 26-II ist mit dem zweiten Anodenspannungsausgang 34i-II, dem zweiten Kathodenspannungsausgang 34g-II und dem Masseanschluss des Röntgenvorschaltgeräts 34* verbunden. Die Röntgenröhren 26-I, 26-II sind hinter der Abschirmung 28 mit einem Fenster (einer Blende) 28a angeordnet.

Je nach Inhalt eines zugeführten Röntgen-Ansteuerungssignals s_{R} stellt das Röntgenvorschaltgerät 34* wahlweise entweder die erste Anodenspannung U_{a,I} und die erste Heizspannung U_{h,I} an dem ersten Anodenspannungsausgang 34i-I und dem ersten Kathodenspannungsausgang 34g-I bereit, aktiviert also die erste Röntgenröhre 26-I (angenommene Situation), oder stellt die zweite Anodenspannung U_{a,II} und die zweite Heizspannung U_{h,II} an dem zweiten Anodenspannungsausgang 34i-II und dem zweiten Kathodenspannungsausgang 34g-II bereit, aktiviert also die zweite Röntgenröhre 26-II. Im ersten Fall sendet die erste Röntgenröhre 26-I einen Röntgenimpuls eines ersten Frequenzbereichs f_{I} in einem Strahlenkegel 36-I aus. Im zweiten Fall sendet die zweite Röntgenröhre 26-II einen Röntgenimpuls eines zweiten Frequenzbereichs f_{II} in einem Strahlenkegel 36-II aus.

Die Strahlenkegel 36-I, 36-II der Röntgenröhren 26-I, 26-II weisen leicht unterschiedliche Ausrichtungen auf; das Diagnoseobjekt (30, vgl. Fig. 4) befindet sich in einem Bereich, der von beiden Strahlenkegeln 36-I, 36-II beleuchtet wird. Insbesondere fallen beide Stahlenkegel 36-I, 36-II auf den CCD-Sensor (32, vgl. Fig. 4). Der Winkelunterschied zwischen den Strahlenkegeln 36-I, 36-II ist bekannt und wird bei der Bildbearbeitung herausgerechnet.

Die Anordnung der Röntgenröhren in Fig. 17 ist rein schematisch zu verstehen. Eine mögliche Schrägstellung der Röntgenröhren 26-I, 26-II ist in der Figur nicht dargestellt. Die Röntgenröhren können sehr dicht beieinander, auch übereinander, angeordnet sein, sodass der Winkelunterschied in den Strahlengängen minimiert sind.

In einer Abwandlung ist in dem Strahlengang der ersten Röntgenröhre 26-I und der zweiten Röntgenröhre 26-II eine Strahlenumlenkeinrichtung angeordnet, die dafür sorgt, dass der Strahlengang der ersten Röntgenröhre 26-I und der zweiten Röntgenröhre 26-II spätestens jenseits der Blende 28a zusammenfallen.

Die Röntgenröhren 26-I, 26-II können von gleicher Bauart sein oder jeweils an bestimmte bevorzugte Frequenzbereiche angepasst sein. So kann z.B. die erste Röntgenröhre 26-I auf Frequenzbereiche bei Beschleunigungsspannungen zwischen 40 und 80 kV angepasst sein, und kann die zweite Röntgenröhre 26-II an Frequenzbereiche bei Beschleunigungsspannungen zwischen 80 und 120 kV angepasst sein.

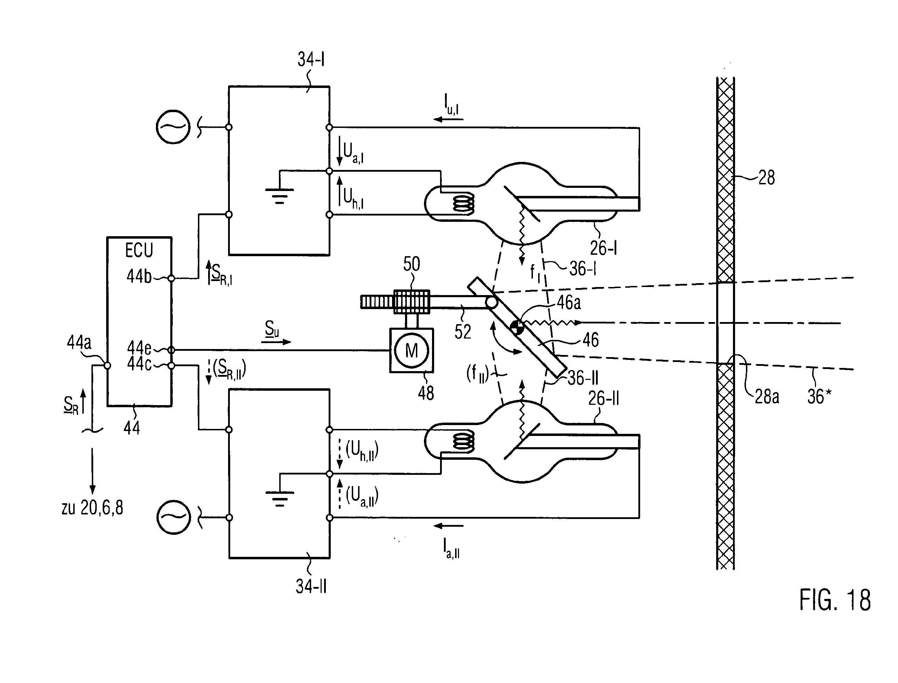

Fig. 18 zeigt schematisch eine weitere Ausführungsvariante mit zwei Röntgenröhren 26-I, 26-II, wobei zwei Röntgenvorschaltgeräte 34-I, 34-II und eine gemeinsame Röntgen-Steuereinheit 44 und eine Strahlrichtungsumschaltung vorgesehen sind.

Die Röntgenröhren 26-I, 26-II und die Röntgenvorschaltgeräte 34-I, 34-II dieser Ausführungsvariante entsprechen in Aufbau, Zusammenschaltung und Funktion grundsätzlich der Röntgenröhre 26 und dem Röntgenvorschaltgerät 34 in Fig. 5, wobei auf eine Umschaltbarkeit der Röntgenvorschaltgeräte 34 verzichtet werden kann. Gleichwohl können die Röntgenvorschaltgeräte 34 ausgelegt sein, einen Bereich verschiedener (wählbarer) Anodenspannungen und/oder Heizspannungen und/oder Röhrenströme abzudecken. Zur Unterscheidung sei angenommen, dass das erste Röntgenvorschaltgerät 34-I eingestellt ist, um auf Zuführung eines ersten Röntgen-Ansteuerungssignals s_{R,I} hin eine erste Anodenspannung U_{a,I} und eine erste Heizspannung U_{h,I} bereitzustellen, und dass das zweite Röntgenvorschaltgerät 34-II eingestellt ist, um auf Zuführung eines zweiten Röntgen-Ansteuerungssignals s_{R,II} hin eine zweite Anodenspannung U_{a,II} und eine zweite Heizspannung U_{h,II} bereitzustellen. Die erste Röntgenröhre 26-I ist mit dem erste Röntgenvorschaltgerät 34-I verbunden, und die zweite Röntgenröhre 26-II ist mit dem zweiten Röntgenvorschaltgerät 34-II verbunden.

Eine elektronische Steuereinheit (ECU) 44 (auch als Röntgen-Steuergerät 44 bezeichnet) ist über einen Steuersignaleingang 44a und das Netzwerk (vgl. Fig. 2) mit der Bedienkonsole 20, dem Server 6 und der Arbeitsstation 8 verbindbar und stellt je nach Inhalt eines zugeführten Röntgen-Ansteuerungssignals s_{R} an einem ersten Steuersignalausgang 44b das erste Röntgen-Ansteuerungssignal s_{R,I} für das erste Röntgenvorschaltgerät 34-I bereit oder stellt an einem zweiten Steuersignalausgang 44c das zweite Röntgen-Ansteuerungssignal S_{R,II} für das zweite Röntgenvorschaltgerät 34-II bereit. Im ersten Fall stellt das Röntgenvorschaltgerät 34-I die erste Anodenspannung U_{a,I} und die erste Heizspannung U_{h,I} bereit, aktiviert also die erste Röntgenröhre 26-I (angenommene Situation). In diesem Fall sendet die erste Röntgenröhre 26-I einen Röntgenimpuls eines ersten Frequenzbereichs f_{I} in einem Strahlenkegel 36-I aus. Im zweiten Fall stellt das Röntgenvorschaltgerät 34-II die zweite Anodenspannung U_{a,II} und die zweite Heizspannung U_{h,II} bereit, aktiviert also die zweite Röntgenröhre 26-II. In diesem Fall sendet die zweite Röntgenröhre 26-II einen Röntgenimpuls eines zweiten Frequenzbereichs f_{II} in einem Strahlenkegel 36-II aus.

Die erste Röntgenröhre 26-I und die zweite Röntgenröhre 26-II sind hinter der Abschirmung 28 mit der Blendenöffnung 28a einander gegenüber angeordnet, sodass ihre Strahlenkegel 36-I, 36-II aufeinander zu laufen. Zwischen der ersten Röntgenröhre 26-I und der zweiten Röntgenröhre 26-II ist ein Reflektor 46 angeordnet, der um eine Achse 46a schwenkbar gelagert ist. Ein Motor 48 treibt mittels eines Ritzels 50, das an seiner Abtriebswelle befestigt ist, eine Zahnstange 52, die über ein Gelenk (nicht näher bezeichnet) mit dem Reflektor 46 verbunden ist. Der Motor 48 ist an einem dritten Steuersignalausgang 44d des Steuergeräts 44 angeschlossen. Über den Steuersignalausgang 44c ist dem Motor 48 ein Umschalt-Ansteuerungssignal s_{U} zuführbar. Je nach Inhalt eines zugeführten Umschalt-Ansteuerungssignals s_{U} fährt der Motor 48 die Zahnstange 52 in eine erste oder eine zweite Endstellung. In der ersten Endstellung der Zahnstange 52 befindet sich der Reflektor 46 in einer ersten Schwenkstellung, in welcher ein Strahlenkegel 36-I der ersten Röntgenröhre 26-I auf die Blende 28a der Abschirmung 28 geworfen wird. In der zweiten Endstellung der Zahnstange 52 befindet sich der Reflektor 46 in einer zweiten Schwenkstellung, in welcher ein Strahlenkegel 36-II der zweiten Röntgenröhre 26-II auf die Blende 28a der Abschirmung 28 geworfen wird. Außerhalb der Blende 28a ist nur noch ein einziger Strahlenkegel 36* feststellbar, der entweder Strahlung des Frequenzbandes f_{I} oder des Frequenzbandes f_{II} aufweist.

Es versteht sich, dass die Darstellung des Reflektors 46 als ebene reflektierende Platte schematisch ist, um die Reflektionseigenschaft zu veranschaulichen. Der Aufbau des Reflektors 46 kann eine andere Geometrie aufweisen. Der Reflektor 46 kann Kristalle aufweisen, die im Bereich der auftretenden Wellenlängen bei einem bestimmten Einfallswinkel (Glanzwinkel) die Bragg-Bedingung erfüllen und den einfallenden Strahl auf das Fenster 28a werfen. Die Endstellungen der Zahnstange 52 sind auf die Reflektionseigenschaften des Reflektors 46 abgestimmt. Die Röntgenquellen 26-I, 26-II können einen Winkel zueinander aufweisen, der sich von dem dargestellten unterscheidet; Position und Winkellage können einstellbar und/oder verfahrbar sein, um eine ideale Lage in Bezug auf den Reflektor 46 aufzuweisen.

In einer Abwandlung ist zwischen der ersten Röntgenröhre 26-I und der zweiten Röntgenröhre 26-II eine feststehende Strahlenlenkeinrichtung angeordnet, die sowohl den Strahlenkegel 36-I der ersten Röntgenröhre 26-I als auch den Strahlenkegel 36-II der zweiten Röntgenröhre 26-II auf die Blende 28a umlenkt, sodass sie zu einem gemeinsamen Strahlenkegel 36* zusammenfallen.

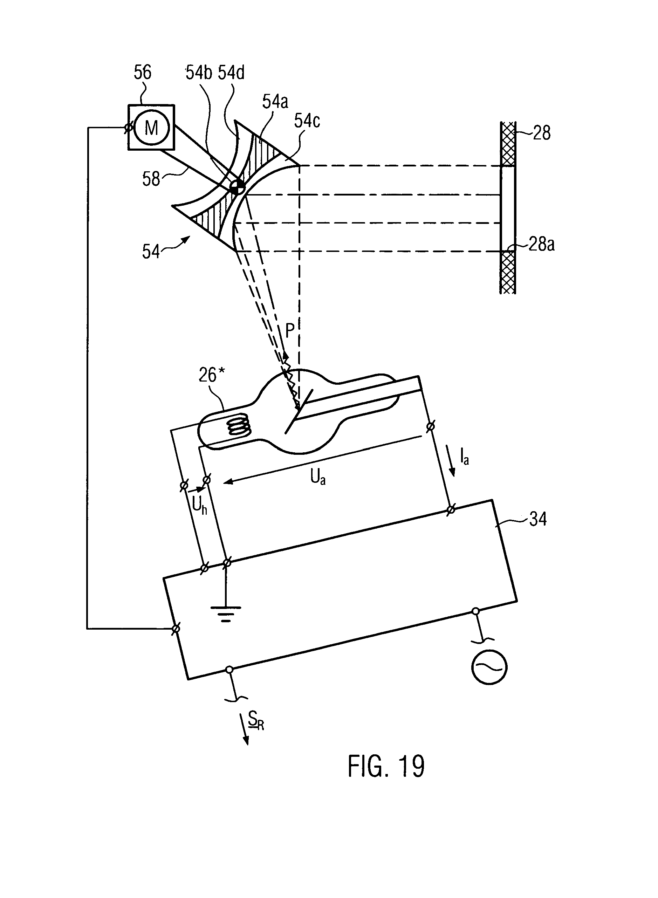

Fig. 19 zeigt schematisch als eine weitere Ausführungsvariante der vorliegenden Erfindung eine Röntgenquelle zur Erzeugung von Röntgenstrahlung zweier unterschiedlicher Spektren.

Bei dieser Ausführungsvariante ist eine Breitband-Röntgenröhre 26* vorgesehen, die bei Anlegen einer Beschleunigungsspannung Uₐ und einer Heizspannung Uₕ Röntgenstrahlung p in einem breiten Frequenzband abgibt. Die Breitband-Röntgenröhre 26* wird von einem Röntgenvorschaltgerät 34 angesteuert (getaktet).

Im Strahlengang der Breitband-Röntgenröhre 26* ist eine Frequenzselektoreinheit 54 angeordnet. Die Frequenzselektoreinheit 54 weist eine Trägerstruktur 54a auf, die um eine Schwenkachse 54b schwenkbar ist und auf deren gegenüberliegenden Seiten eine erste Monochromatorstruktur 54c bzw. eine zweite Monochromatorstruktur 54d angebracht sind. Die Trägerstruktur 54a ist um die Schwenkachse 54b derart schwenkbar, dass in einer ersten Schwenkstellung die erste Monochromatorstruktur 54c und in einer zweiten Schwenkstellung die zweite Monochromatorstruktur 54d der Röntgenröhre zugewandt ist. Die erste Monochromatorstruktur 54c und die zweite Monochromatorstruktur 54d weisen jeweils einen sogenannten Bragg-Kristall auf. Ein Bragg-Kristall ist ein Kristall, der unter einem bestimmten Einfallswinkel, Glanzwinkel genannt, z.B. Röntgenstrahlung einer bestimmten Wellenlänge aufgrund von Interferenzerscheinungen unter einem bestimmten Ausfallswinkel reflektiert, während Strahlung anderer Wellenlängen und unter anderen Winkeln ausgelöscht oder in anderen Richtungen reflektiert wird. Ein Intensitätsmaximum der reflektierten Strahlung wird durch die sog. Bragg-Bedingung defininiert, die den Einfallswinkel mit der Wellenlänge in Beziehung setzt. Das Prinzip der Bragg-Reflektion (Bragg'sche Bedingung) ist wohlbekannt und muss hier nicht im Einzelnen ausgeführt werden. Die Monochromatorstrukturen 54c, 54d weisen eine konkav geschliffene Form auf, welche dafür sorgt, dass reflektierte Strahlung in im Wesentlichen paralleler Richtung ausfällt.

Ein Schwenkmotor 56 ist über einen Riemen 58 mit der Schwenkachse 54b der Frequenzselektoreinheit 54 verbunden und über das Röntgenvorschaltgerät 34 im Takt der Röntgenimpulse ansteuerbar, um die Frequenzselektoreinheit 54 in die erste oder die zweite Schwenkstellung zu schwenken. Die erste und die zweite Schwenkstellung und die Anordnung und Form der Monochromatorstrukturen 54c, 54d sind so ausgelegt, dass Röntgenstrahlung jeweils in Richtung des Blendenfensters 28a der Abschirmung 28 geworfen wird. Unerwünschte Röntgenstrahlung wird in der Abschirmung 28 aufgefangen. Auf diese Weise kann mit einer einzigen Röntgenröhre Röntgenstrahlung mit zwei festgelegten Frequenzspektren erzeugt werden.

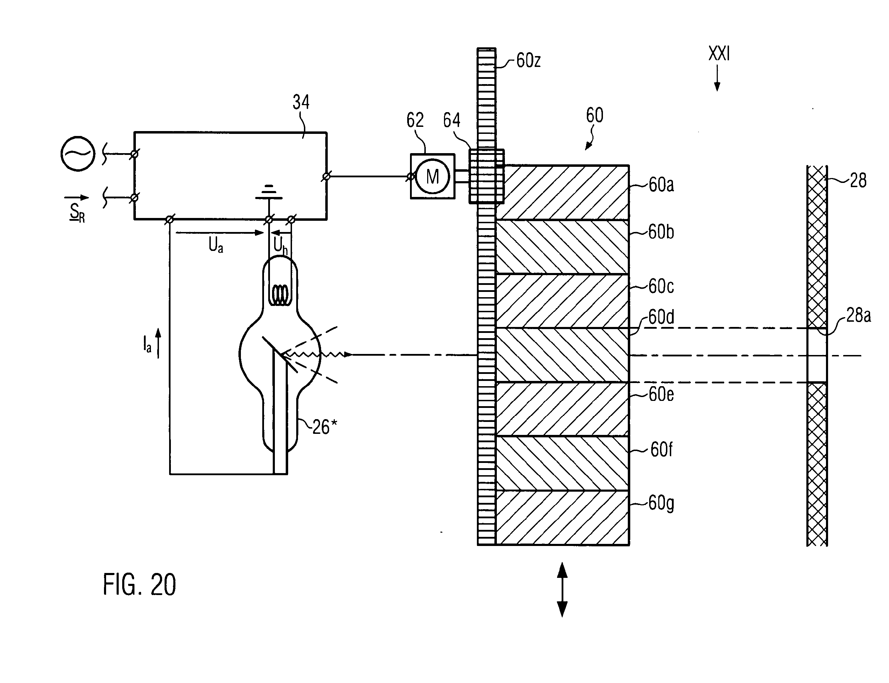

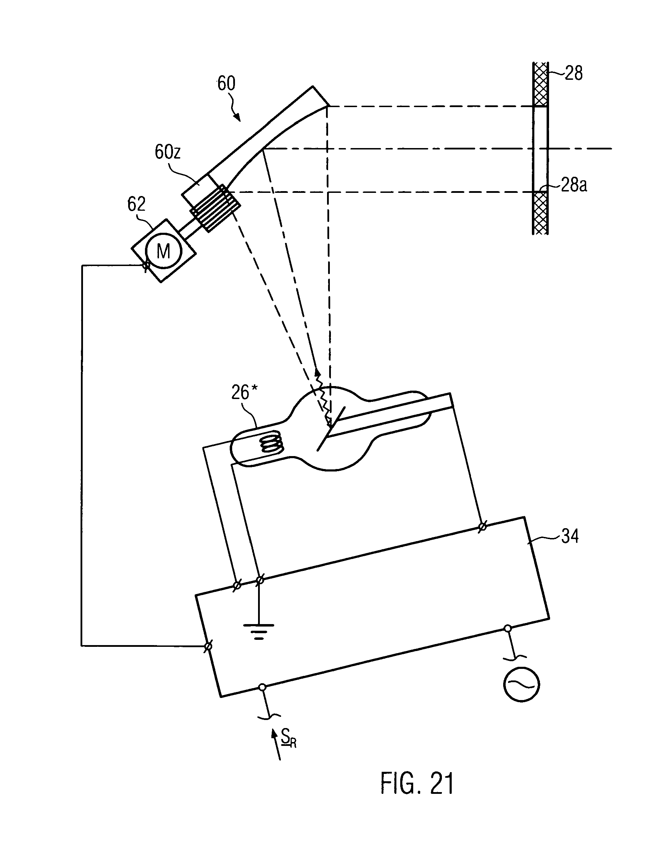

Fig. 20 und Fig. 21 zeigen schematisch als eine weitere Ausführungsvariante der vorliegenden Erfindung eine Röntgenquelle zur Erzeugung von Röntgenstrahlung mehrerer unterschiedlicher Spektren. Die Darstellung in Fig. 20 ist so gewählt, dass die Blickrichtung im Wesentlichen senkrecht auf die Oberfläche von Monochromatorstrukuren weist und der Strahlengang einer Röntgenröhre gestreckt dargestellt ist. Die Blickrichtung in Fig. 21 entspricht einem Pfeil XXI in Fig. 20.

Wie bei der vorherigen Ausführungsvariante ist eine Breitband-Röntgenröhre 26* vorgesehen, die bei Anlegen einer Beschleunigungsspannung Uₐ und einer Heizspannung Uₕ Röntgenstrahlung p in einem breiten Frequenzband abgibt. Die Breitband-Röntgenröhre 26* wird von einem Röntgenvorschaltgerät 34 angesteuert (getaktet).

Im Strahlengang der Breitband-Röntgenröhre 26* ist eine Frequenzselektoreinheit 60 angeordnet. Die Frequenzselektoreinheit 60 weist eine Mehrzahl von übereinander angeodneten Monochromatorstrukturen 60a, 60b, ... 60g auf. Die gesamte Struktur ist mittels einer Zahnstange 60z, eines Stellmotors 62 und eines in die Zahnstange eingreifenden Ritzels 64 in Richtung eines Doppelpfeils in der Figur linear verfahrbar. Der Stellmotor 62 ist über das Röntgenvorschaltgerät 34 ansteuerbar.

Die Monochromatorstrukturen 60a, 60b, ... 60g weisen jeweils einen Bragg-Kristall auf. Hinsichtlich Aufbau und Funktionsweise der Monochromatorstrukturen 60a, 60b, ... 60g wird auf die vorstehenden Ausführungen zu den Monochromatorstrukturen 54c, 54d der Ausführungsvariante gemäß Fig. 19 Bezug genommen.

Auf Zuführen eines Röntgen-Ansteuerungssignals s_{R} hin steuert das Röntgenvorschaltgerät 34 den Stellmotor 62 an, um die Frequenzselektoreinheit 60 in eine Position derart zu verfahren, dass die passende Monochromatorstruktur 60a, 60b, ... 60g sich im Strahlengang von der Breitband-Röntgenröhre 26* zu dem Fenster 28a der Abschirmung 28 befindet, und pulst die Breitband-Röntgenröhre 26* mit der Beschleunigungsspannung Uₐ und der Heizspannung Uₕ. Die von der Breitband-Röntgenröhre 26* ausgesendete Strahlung wird durch die im Strahlengang befindliche Monochromatorstruktur in Richtung des Blendenfensters 28a der Abschirmung 28 geworfen. Unerwünschte Röntgenstrahlung wird in der Abschirmung 28 aufgefangen. Auf diese Weise kann mit einer einzigen Röntgenöhre Röntgenstrahlung mit unterschiedlichen festgelegten Frequenzspektren erzeugt werden.

Der Erfinder hat das vorstehend beschriebene Verfahren der Erfindung an einem Volumentomographen mit Röntgenspektren getestet, die bei einer ersten Beschleunigungsspannung von 60 kV und einem ersten Röhrenstrom von 10 mA einerseits und einer zweiten Beschleunigungsspannung von 84 kV und einem zweiten Röhrenstrom von 12 mA erzeugt wurden. Bei Aufnahmen, die mit einem Volumentomograohen mit Flächendetektor mit einem bestimmten Frequenzspektrum erzeugt werden, wird konstruktionsbedingt ein im Vergleich mit einem Spiraltomographen (CT) geringer Bildkontrast erreicht. Durch Bildüberlagerung der primären Röntgendaten mit unterschiedlichen Röntgenspektren gemäß einem der vorstehenden Verfahren konnte dieser Nachteil deutlich gemindert werden. Die verwendeten Spektren können an die jeweiligen Erfordernisse angepasst werden.

Bisher werden DVT-Geräte fast ausschließlich im Kopfbereich eingesetzt, da sie bei relativ gerninger Strahlenbelastung ein dreidimensionales Bild in annehmbarer Qualität ergeben. Durch die vorliegende Erfindung kann die Qualität der Bilder derart verbessert werden, dass ein Einsatz auch bei Gliedmaßen, z.B. am Knie und am Sprunggelenk, sinnvoll wird. Derartige Untersuchungen waren bisher nur mit Magnetresonanztomographen möglich. Dennoch muss die Strahlenbelastung nicht höher als bei den herkömmlichen Verfahren sein.

Bei der Beschreibung der bevorzugten Ausführungsformen, Ausführungsvarianten und Abwandlungen wurde davon ausgegangen, dass das Diagnoseobjekt im Wesentlichen raumfest angeordnet ist und sich die Röntgenfunktionseinheit um das Diagnoseobjekt herum bewegt. Insbesondere, aber nicht nur, bei unbelebten Diagnoseobjekten, etwa in der Pathologie,der Archäologie, der Paläontologie oder bei materialwissenschaftlichen Untersuchungen kann das Funktionsprinzip auch umgekehrt werden und bei feststehender Röntgenfunktionseinheit das Diagnoseobjekt bewegt werden.

Die beschriebenen Ausführungsvarianten der Erfindung beinhalten mit geringfügigen Anpassungen auch Anwendungen, bei denen mehr als nur zwei unterschiedliche Frequenzspektren verwendet und die damit erzeugten Einzelbilder

Es ist denkbar, mit geringfügigen Anpassungen die Lehre der Erfindung auch auf Strahlung anderer Frequenzbereiche, die zur Bildgebung ausgenutzt werden, anzuwenden. Beispiele für Strahlungsarten, die zur Bildgebung ausgenutzt werden, sind Mikrowellen, Terahertzwellen, harte Röntgenstrahlung im Bereich von beispielsweise 160 bis 200 kV, Infrarotstrahlung, Schallwellen (Ultraschall) oder dergleichen. Ein Einsatz ist auch bei passiven Bildgebungsverfahren (Nuklearmedizin) denkbar.

Hinsichtlich vorstehend im einzelnen nicht näher erläuterter Merkmale der Erfindung wird im übrigen ausdrücklich auf die Patentansprüche und die Zeichnung verwiesen.

### Bezugszeichenliste

- 2: DVT-System
- 4: DVT-Gerät
- 6: Server
- 8: Arbeitsstation
- 8a: Tastatur
- 8b: Bildschirm
- 10: Säule
- 12: Läufer
- 14: Ausleger
- 16: C-Arm
- 16a: Basis
- 16b: erster Arm (Belichtungsarm)
- 16c: zweiter Arm (Sensorarm)
- 18: Positionierungstisch
- 18a: Handgriffbügel
- 18b: Kinnauflage
- 18c: Stirnstütze
- 20: Bedienkonsole
- 22: Not-Aus-Schalter
- 24: Motor
- 26, 26-I, 26-II: Röntgenröhre
- 26*: Breitband-Röntgenröhre
- 26a: Kolben
- 26b: Heizwendel (Kathode)
- 26c: Anode
- 26d: Wehnelt-Zylinder
- 28: Abschirmung
- 28a: Blende
- 30: Objekt
- 32: CCD-Sensor
- 34, 34*, 34-I, 34-II: Röntgen-Vorschaltgerät
- 34a: Hochspannungsquelle
- 34b: CPU
- 34c: Anodenschaltnetz
- 34d: Netzanschluss
- 34e: Signaleingang
- 34f: Masseausgang
- 34g: Heizspannungsausgang
- 34h: Anodenspannungsausgang
- 34i: Kathodenschaltnetz
- 36, 36-I, 36-II, 36*: Strahlenkegel
- 38: Referenzobjekt (Kugel)
- 40: Schutzglas
- 42: Referenzobjekt (Foliendreieck)
- 44: Röntgen-Steuergerät
- 44a: Steuersignaleingang
- 44b, 44c, 44d: Steuersignalausgänge
- 46: Reflektor
- 46a: Schwenkachse
- 48: Motor
- 50: Ritzel
- 52: Zahnstange
- 54: Frequenzselektoreinheit
- 54a: Trägerstruktur
- 54b: Schwenkachse
- 54c: 1. Monochromatorstruktur
- 54d: 2. Monochromatorstruktur
- 56: Schwenkmotor
- 58: Riemen
- 60: Frequenzselektoreinheit
- 60a, 60b, ... 60g: Monochromatorstruktur
- 60z: Zahnstange
- 62: Stellmotor
- 64: Ritzel

- e: Elektron
- i: Laufvariable
- f: Frequenz(-band)
- n: Anzahl von Inkrementschritten
- p: Photon
- s_{M}: Motor-Ansteuerungssignal
- s_{P}: Positionssignal
- s_{R}: Röntgenquellen-Ansteuerungssignal
- s_{U}: Umschalt-Ansteuerungssignal
- u,v: Koordinaten eines Einzelbildes
- w: Drehwinkel bzw. Winkellage (Sollwert)
- w₀: Winkelinkrement
- w₁: Anfangswinkel
- w₂: Endwinkel
- x,y,z: Raumrichtungen

- dG: Grauwertdifferenz
- Eᵢ(u,v): Einzelbild
- Fᵢ(u,v): Gefiltertes Einzelbild
- F(x,y,z): Gefiltertes Raumbild
- G: Mittlerer Grauwert
- I,II: Kennziffern für Frequenzbänder
- Iₐ: Röhrenstrom (Anodenstrom)
- Nᵢ(u,v): Normalisiertes Einzelbild
- N(x,y,z): Normalisiertes Raumbild
- R(x,y,z): Raumbild (Rekonstruktion)
- R1, R2: Widerstände
- S1, S2: Schalter
- S(x,y,z): Gesamt-Raumbild (Überlagerung)
- U₀,V₀: Anzahl der Spalten und Zeilen eines Röntgensensors
- Uₐ: Anodenspannung (Beschleunigungsspannung)
- Uₕ: Heizspannung (Kathodenspannung)
- Uₓ: Hochspannung
- W: Winkelbereich
- X: Gewichtungsfaktor
- X₀,Y₀,Z₀: Maximale Ausdehnung des diagnostizierten Raumbereichs in Voxel-Einheiten

Die vorliegende Liste der Bezugszeichen und Formelzeichen ist integraler Bestandteil der Beschreibung.

## Patentansprüche

1. Verfahren zur digitalen Volumentomographie an einem Objekt, wobei eine Röntgenfunktionseinheit mit einer Röntgenquelle und einer digitalen, zweidimensionalen Röntgendetektoreinrichtung und einem dazwischen sich erstreckenden Raumvolumen, in welchem sich das Objekt befindet, gedreht wird und dabei einen vorbestimmten Winkelbereich überstreicht, wobei die Röntgenquelle in diskreten, vorbestimmten Winkelabständen angesteuert wird, um Röntgenstrahlen auszusenden, wobei die durch die Detektoreinrichtung aufgefangenen Signale in Zuordnung zu der jeweiligen Winkellage als zweidimensionale Einzelbilder gespeichert werden, und wobei aus den Einzelbildern eine dreidimensionale Darstellung des abgetasteten Raumvolumens rekonstruiert wird, wobei Röntgenstrahlen wenigstens zweier unterschiedlicher Frequenzbereiche verwendet werden, **dadurch gekennzeichnet, dass** ein Grauwertabgleich (S980) während einer Bildbearbeitung oder der Rekonstruktion durchgeführt wird, wobei Unterschiede in der Gesamthelligkeit von in unterschiedlichen Frequenzbereichen aufgenommenen Bildern ausgeglichen werden, und nach der Rekonstruktion der dreidimensionalen Darstellung des abgetasteten Raumvolumens zwei der mit unterschiedlichen Frequenzbereichen aufgenommenen dreidimensionalen Darstellungen überlagert werden (S990), derart, dass die zu dem Objekt gehörenden Strukturen in den beiden dreidimensionalen Darstellungen zusammenpassen.

2. Verfahren gemäß Anspruch 1, **dadurch gekennzeichnet, dass** der vorbestimmte Winkelbereich in wenigstens zwei Durchgängen von der Röntgenfunktionseinheit überstrichen wird, wobei bei jedem Durchgang ein anderer Frequenzbereich der Röntgenquelle verwendet wird, um Einzelbilder zu erzeugen, und wobei die Einzelbilder jedes Durchgangs zu einer räumlichen Darstellung rekonstruiert werden, wobei der Grauwertabgleich zwischen den räumlichen Darstellungen auf der Grundlage eines mittleren Grauwerts der Voxel jeder räumlichen Darstellung durchgeführt wird, und wobei die räumlichen Darstellungen nach dem Grauwertabgleich überlagert werden, um eine einzige räumliche Darstellung zu erzeugen.

3. Verfahren gemäß Anspruch 1, **dadurch gekennzeichnet, dass** die Überlagerung mit Hilfe eines Stitching-Verfahrens durchgeführt wird.

4. Verfahren gemäß Anspruch 2 oder 3, **dadurch gekennzeichnet, dass** die Drehrichtung nach jedem Überstreichen des vorbestimmten Winkelbereichs wechselt.

5. Verfahren gemäß einem der Ansprüche 1 bis 4, **dadurch gekennzeichnet, dass** der Grauwertabgleich anhand eines Referenzkörpers durchgeführt wird, der in der Nähe oder innerhalb des Objekts im Strahlengang der Röntgenfunktionseinheit angeordnet ist.

6. Verfahren gemäß Anspruch 5, **dadurch gekennzeichnet, dass** der Referenzkörper aus einem Kunststoff hergestellt ist, der vorzugsweise mit einer Röntgenkontrastsubstanz, enthaltend beispielsweise Bariumsulfat, Eisen, Titan oder dergleichen, besonders bevorzugt als Nanopartikel, derart dotiert oder beschichtet ist, dass eine Wechselwirkung von Röntgenstrahlung mit dem Referenzkörper einer Wechselwirkung mit einer Materialart, welche in dem Objekt enthalten ist, ähnlich ist.

7. Verfahren gemäß Anspruch 1, **dadurch gekennzeichnet, dass** der vorbestimmte Winkelbereich in einem einzigen Durchgang durchlaufen wird, wobei der Fequenzbereich der Röntgenquelle nach jedem Winkelinkrement gewechselt wird, wobei ein Grauwertabgleich zwischen benachbarten Einzelbildern auf der Grundlage eines mittleren Grauwerts der Pixel jedes Einzelbildes durchgeführt wird.

8. Verfahren gemäß Anspruch 7, **dadurch gekennzeichnet, dass** der Grauwertabgleich anhand eines Referenzkörpers durchgeführt wird, der in der Nähe der Röntgendetektoreinrichtung mitdrehend im Strahlengang der Röntgenfunktionseinheit angeordnet ist.

9. Verfahren gemäß Anspruch 8, **dadurch gekennzeichnet, dass** der Referenzkörper aus einem Kunststoff hergestellt ist, der vorzugsweise mit einer Röntgenkontrastsubstanz, enthaltend beispielsweise Bariumsulfat, Eisen, Titan oder dergleichen, besonders bevorzugt als Nanopartikel, derart dotiert oder beschichtet ist, dass eine Wechselwirkung von Röntgenstrahlung mit dem Referenzkörper einer Wechselwirkung mit einer Materialart, welche in dem Objekt enthalten ist, ähnlich ist.

10. Verfahren gemäß Anspruch 8, **dadurch gekennzeichnet, dass** der Referenzkörper ein beschichtets Folienstück, beispielsweise Foliendreieck, ist, das auf der Oberfläche der Röntgendetektoreinrichtung vorzugsweise im Randbereich, möglichst ohne das Objekt zu verdecken, aufgebracht ist.

11. Vorrichtung zur digitalen Volumentomographie, aufweisend eine drehbare Röntgenfunktionseinheit mit eine Röntgenquelle und eine digitalen Detektoreinrichtung, wobei die Röntgenquelle eingerichtet und angepasst ist, Röntgenstrahlen verschiedener Frequenzbereiche abzugeben, und eine Steuereinrichtung, die eingerichtet und angepasst ist, eine Antriebseinrichtung anzusteuern, um die Röntgenfunktionseinheit zu drehen und eine vorgegebene Winkellage einzustellen, die Röntgenquelle in diskreten Winkelinkrementen mehrmals anzusteuern, um Röntgenstrahlung abzugeben, und die von der Detektoreinrichtung aufgefangenen Signale als zweidimensionale Einzelbilder zu speichern und einer bestimmten Winkellage zuzuordnen, und aus den Einzelbildern eine dreidimensionale Darstellung zu berechnen, **dadurch gekennzeichnet, dass** die Vorrichtung eingerichtet und angepasst ist, ein Verfahren nach einem der vorstehenden Ansprüche durchzuführen.

12. Vorrichtung nach Anspruch 11, **dadurch gekennzeichnet, dass** die Röntgenquelle zwei Röntgenröhren aufweist, die in unterschiedlichen Frequenzbereichen arbeiten und die wahlweise aktivierbar sind.

13. Vorrichtung nach Anspruch 11, **dadurch gekennzeichnet, dass** die Röntgenquelle eine breitoder mehrbandige Röntgenröhre sowie eine Frequenzselektionseinrichtung, die ausgelegt und eingerichtet ist, unterschiedliche Frequenzbereiche wahlweise durchzulassen, aufweist.

14. Vorrichtung nach Anspruch 13, **dadurch gekennzeichnet, dass** die Frequenzselektionseinrichtung wenigstens einen Bragg-Kristall auf, der mit veränderbarer Lage in dem Strahlengang platziert ist und/oder, falls mehrere Bragg-Kristalle vorhanden sind, die wahlweise in dem Strahlengang platzierbar sind.

15. Vorrichtung nach einem der Ansprüche 11 bis 14, **dadurch gekennzeichnet, dass** die Verbindung zwischen der Röntgenquelle, der Röntgendetektoreinrichtung und dem Drehantrieb einerseits und der Steuerungseinrichtung andererseits drahtgebunden und/oder drahtlos, insbesondere über Bluetooth, WLAN, eine Infrarotverbindung und/oder dergleichen herstellbar ist.

## Claims

1. Method of digital volume tomography of an object, wherein an X-ray functional unit with an X-ray source and a digital, two-dimensional X-ray detector unit, and a volume of space extending between them in which the object is located, are rotated and in the process a predetermined angular range is overswept, wherein the X-ray source is actuated at discrete, predetermined angular distances in order to emit X-rays, wherein the signals picked up by the detector unit are stored as two-dimensional images assigned to the respective angular position, and wherein from the single images a three-dimensional representation of the scanned volume of space is reconstructed, wherein X-rays of at least two different frequency ranges are used, **characterised in that** a grey-scale calibration (S980) is made during an image processing or reconstruction, wherein differences in the overall brightness of images taken in different frequency ranges are compared, and after reconstruction of the three-dimensional representation of the scanned volume of space, two of the three-dimensional representations taken with different frequency ranges are superimposed (S990) in such a way that the structures in the two three-dimensional representations belonging to the object fit together.

2. Method according to claim 1, **characterised in that** the predetermined angular range is overswept by the X-ray functional unit in at least two passes, wherein for each pass a different frequency range of the X-ray source is used, in order to make single images, and wherein the single images of each pas are reconstructed to make a three-dimensional representation, wherein the grey-scale calibration between the three-dimensional representations is made on the basis of an average grey-scale value of the voxels of each three-dimensional representation, and wherein the three-dimensional representations are superimposed according to the grey-scale calibration, in order to create a single three-dimensional representation.

3. Method according to claim 1, **characterised in that** the superimposition is made with the aid of a stitching process.

4. Method according to claim 2 or 3, **characterised in that** the direction of rotation is changed after each sweep over the predetermined angular range.

5. Method according to any of claims 1 to 4, **characterised in that** the grey-scale calibration is made with the aid of a reference body which is arranged in the vicinity of or within the object in the beam path of the X-ray functional unit.

6. Method according to claim 5, **characterised in that** the reference body is made of plastic, preferably doped or coated with an X-ray contrast substance containing for example barium sulphate, iron, titanium or the like, especially preferably in the form of nanoparticles, in such a way that an interaction of X-radiation with the reference body is similar to an interaction with a material which is contained in the object.

7. Method according to claim 1, **characterised in that** the predetermined angular range is traversed in a single pass, wherein the frequency range of the X-ray source is changed after each angular increment, wherein a grey-scale calibration between adjacent single images is made on the basis of an average grey-scale value of the pixels of each single image.

8. Method according to claim 7, **characterised in that** the grey-scale calibration is made with the aid of a reference body which is arranged in the vicinity of the X-ray detector unit, also rotating in the beam path of the X-ray functional unit.

9. Method according to claim 8, **characterised in that** the reference body is made of plastic, preferably doped or coated with an X-ray contrast substance containing for example barium sulphate, iron, titanium or the like, especially preferably in the form of nanoparticles, in such a way that an interaction of X-radiation with the reference body is similar to an interaction with a material which is contained in the object.

10. Method according to claim 8, **characterised in that** the reference body is a coated piece of film, for example a triangle of film, which is applied to the surface of the X-ray detector unit, preferably in the edge area, if possible without covering the object.

11. Apparatus for digital volume tomography, having a rotatable X-ray functional unit with an X-ray source and a digital detector unit, wherein the X-ray source is set up and adapted to emit X-rays in different frequency ranges, and a control unit which is set up and adapted to control a drive unit in order to rotate the X-ray functional unit and to set a preset angular position, to control the X-ray source several times in discrete angular increments in order to emit X-radiation, and to store the signals picked up by the detector unit as two-dimensional single images and to assign a specific angular position, and to calculate from the single images a three-dimensional representation, **characterised in that** the apparatus is set up and adapted to implement a method according to any of the preceding claims.

12. Apparatus according to claim 11, **characterised in that** the X-ray source has two X-ray tubes which operate in different frequency ranges and may be activated alternately.

13. Apparatus according to claim 11, **characterised in that** the X-ray source has a broadband or multi-band X-ray tube and also a frequency selection device, which is designed and set up to let through different frequency ranges alternately.

14. Apparatus according to claim 13, **characterised in that** the frequency selection device has at least one Bragg crystal which is placed with variable position in the beam path and/or, if several Bragg crystals are provided, they may be placed alternately in the beam path.

15. Apparatus according to any of claims 11 to 14, **characterised in that** the connection between the X-ray source, the X-ray detector unit and the rotary drive on the one hand, and the control unit on the other hand, is wired and/or wireless, in particular may be created via Bluetooth. WLAN, an infrared link and/or the like.

## Revendications

1. Procédé pour la tomographie volumétrique numérique sur un objet, dans lequel une unité fonctionnelle de radiographie avec une source de rayons X et un dispositif de détection de rayons X numérique à deux dimensions et un volume d'espace s'étendant entre ceux-ci, dans lequel se trouve l'objet, est tournée en balayant une plage angulaire prédéterminée, la source de rayons X étant commandée en des écarts angulaires prédéterminés et discrets, afin d'émettre des rayons X, les signaux recueillis par le dispositif de détection étant mémorisés en tant qu'images distinctes à deux dimensions associées à la position angulaire respective, et une représentation tridimensionnelle du volume d'espace balayé étant reconstruit à partir des images distinctes, des rayons X d'au moins deux bandes de fréquences différentes étant utilisées, **caractérisé en ce qu'**un alignement de valeurs de gris (S980) est effectué durant un traitement d'image ou la reconstruction, des différences de luminosité d'images prises en bandes de fréquences différentes étant compensés, et, après la reconstruction de la représentation tridimensionnelle du volume d'espace balayé, deux des représentations tridimensionnelles prises en bandes de fréquences différentes étant superposés (S990) de telle manière, que les structures appartenant à l'objet s'ajustent les uns aux autres dans les deux représentations tridimensionnelles.

2. Procédé selon la revendication 1, **caractérisé en ce que** la plage angulaire prédéterminée est balayée en au moins deux passages par l'unité fonctionnelle de radiographie, une autre bande de fréquence de la source de rayons X étant utilisée durant chaque passage, afin de produire des images distinctes, et les images distinctes de chaque passage étant reconstruites en une représentation spatiale, l'alignement de valeurs de gris entre les représentations spatiales étant effectué sur la base de la valeur de gris moyenne des voxels de chaque représentation spatiale, et les représentations spatiales étant superposées après l'alignement de valeurs de gris, afin de produire une seule représentation spatiale.

3. Procédé selon la revendication 1, **caractérisé en ce que** la superposition est effectuée à l'aide d'un procédé d'assemblage numérique.

4. Procédé selon la revendication 2 ou 3, **caractérisé en ce que** la direction de rotation change après chaque balayage de la plage angulaire prédéterminée.

5. Procédé selon l'une des revendications 1 à 4, **caractérisé en ce que** l'alignement de valeurs de gris est effectué par le biais d'un corps de référence, qui est disposé près ou à l'intérieur de l'objet sur le trajet optique de l'unité fonctionnelle de radiographie.

6. Procédé selon la revendication 5, **caractérisé en ce que** le corps de référence est préparé à partir d'une matière plastique, préférablement dotée de ou revêtue par une substance de contraste pour radiographie comprenant par exemple le sulfate de baryum, le fer, le titane ou similaire, particulièrement préféré en tant que nanoparticule, de telle manière, qu'une interaction de rayons X avec le corps de référence ressemble à une interaction avec une sorte de matériau contenue dans l'objet.

7. Procédé selon la revendication 1, **caractérisé en ce que** la plage angulaire prédéterminée est balayée en un seul passage, la bande de fréquence de la source de rayons X étant changée après chaque incrément angulaire, un alignement de valeurs de gris entre des images distinctes voisines étant effectué à base d'une valeur de gris moyenne des pixels de chaque image distincte.

8. Procédé selon la revendication 7, **caractérisé en ce que** l'alignement de valeurs de gris est effectué par le biais d'un corps de référence, qui est disposé près du dispositif de détection de rayons X de façon à tourner simultanément dans le trajet optique de l'unité fonctionnelle de radiographie.

9. Procédé selon la revendication 8, **caractérisé en ce que** le corps de référence est préparé à partir d'une matière plastique, préférablement dotée avec ou revêtue d'une substance de contraste pour radiographie, contenant par exemple le sulfate de baryum, le fer, le titane ou similaire, particulièrement préféré en tant que nanoparticule, de telle manière, qu'une interaction de rayons X avec le corps de référence ressemble à une interaction avec une sorte de matériaux, contenue dans l'objet.

10. Procédé selon la revendication 8, **caractérisé en ce que** le corps de référence est une partie de feuille revêtue, par exemple un triangle de feuille, qui est appliqué sur la surface du dispositif de détection de rayons X, préférablement dans la zone de bord, sans dissimiler l'objet tant que possible.

11. Dispositif pour la tomographie volumétrique numérique comprenant une unité fonctionnelle de radiographie rotative avec une source de rayons X et un dispositif de détection numérique, la source de rayons X étant configurée et adaptée à produire des rayons X de bandes de fréquences différentes, et un dispositif de commande, qui est configuré et adapté à commander un dispositif d'entraînement afin de tourner l'unité fonctionnelle de radiographie et de régler une position angulaire prédéterminée, à commander la source de rayons X par incréments angulaires discrets à plusieurs reprises, afin d'émettre des rayons X, et à mémoriser les signaux détectés par le dispositif de détection en tant qu'images distinctes en deux dimensions associés à une certaine position angulaire, et à calculer une représentation tridimensionnelle à partir des images distinctes, **caractérisé en ce que** le dispositif est configuré et adapté pour effectuer un procédé selon l'une des revendications précédentes.

12. Dispositif selon la revendication 11, **caractérisé en ce que** la source de rayons X comprend deux tubes de rayons X, qui travaillent en bandes de fréquences différentes et qui peuvent être activé aux choix.

13. Dispositif selon la revendication 11, **caractérisé en ce que** la source de rayons X comprend un tube de rayons X à plusieurs bandes ou à large bande ainsi qu'un dispositif de choix de fréquence, qui est adapté et configuré afin de laisser passer des bandes de fréquences différentes au choix.

14. Dispositif selon la revendication 13, **caractérisé en ce que** le dispositif de choix de fréquence comprend au moins un cristal de Bragg, qui est placé dans le trajet optique en une position pouvant être changée et/ou, si plusieurs cristaux de Bragg sont présents, pouvant être placé aux choix dans le trajet optique.

15. Dispositif selon l'une des revendications 11 à 14, **caractérisé en ce que** la liaison entre la source de rayons X, le dispositif de détection de rayons X et l'entraînement rotatif d'une part et le dispositif de commande d'autre part peut être effectuée par fil ou sans fil, particulièrement par Bluetooth, un réseau local sans fil (WLAN), une connexion infrarouge et/ou similaire.
